# EUROPEAN PATENT APPLICATION

(11) **EP 0 825 262 A2**
(43) Date of publication of application: **25.02.1998**
(21) Application number: 97115220.2
(22) Date of filing: 20.12.1990
(51) Int. Cl.: C12N 15/82, C12N 15/29, A01H 5/00

(54) **Tissue-specific toxin expression in plants**

(30) Priority: 29.12.1989 US 459069; 04.06.1990 US 532907
(62) Divisional of application: 90811009.1
(71) Applicant: Novartis AG, 4058 Basel (CH)
(72) Inventor: Nasrallah, June B., Ithaca, NY 14850 (US); Nasrallah, Mikhail E., Ithaca, NY 14850 (US); Miflin, Benjamin J., London N1 0QY (GB); Thorsness, Mary K., Ithaca, NY 14850 (US)
(74) Representative: Roth, Bernhard M.

(57) **Abstract**

The present invention describes a tissue-specific promoter directing expression of a linked toxin gene in the reproductive tissue of a recipient plant.

SEQUENCE LISTING

(1) GENERAL INFORMATION:

(i) APPLICANT:
(A) NAME: NOVARTIS AG
(B) STREET: Schwarzwaldallee 215
(C) CITY: Basel
(E) COUNTRY: Switzerland
(F) POSTAL CODE (ZIP): 4058
(G) TELEPHONE: +4161 324 11 11
(H) TELEFAX: + 4161 322 75 32

(ii) TITLE OF INVENTION: Tissue-specific toxin expression in plants

(iii) NUMBER OF SEQUENCES: 7

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(2) INFORMATION FOR SEQ ID NO: 1:

(i) SEQUENCE CHARACTERISTICS:
(A) LENGTH: 2336 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: NO

(iii) ANTI-SENSE: NO

(vi) ORIGINAL SOURCE:
(A) ORGANISM: Brassica oleracea var acephala

(ix) FEATURE:
(A) NAME/KEY: CDS
(B) LOCATION: 776..2080
(D) OTHER INFORMATION: /product= "SLG-13
self-incompatibility gene"

(xi) SEQUENCE DESCRIPTION: SEQ ID NO: 1:

GAGCTCTCTG TGCTATTAGA ACATTTATCG TTCATAACCT GACAAATCAA ACGTCCTAAA 60

ACAATACAAA TTTTCATTTT GCAGAACACC AAACGAGTTG TTCGAGATAG TCCCATCATA 120

GGTATAACTT AAAGATTGAC GAATAGGAGT TGAGAAACTG GAAACAAGGG CTTGAGTTAC 180

GATATATCGG AGCGGCTGGG AGCATGTCAC TCGATTTGGC TTGTTTATAG GTTGATTGAT 240

TAAATAAAAC AAAAGCATAT AGTTCGCCGG CCCGGTTGTT GCAACCTTCC AAGGAACGCC 300

TGCTGAGATG GATTTACAAT TTGATTTCTT TTGTATTTTT ATTTGGTGTG TTTAATATAT 360

TAGTTAACCA ATTTACGTTA TACCAAATTT TTCAACCCTC TTTTTAGTAA AAAACGAAAT 420

TAAAGTTTTT TCCCTCTTAG TCCGACGATT TTAAGCTAAT TAGTTCGAAC AAAGAGTACA 480

ACATTAATTT TCTAACAGAC TTAGATGCAC TTGCGAACAA CATACTTGCT GAACACCATA 540

TGTTATGTTG GCAGGGTGAG AAATTAATCA CGTGTAGATA TAGAAGTAGT AGACAAATGA 600

TATAGGTTTG TGGGAATGAA TTAATCGATG GGATGAAAAA GTCATCGAAC ATGTAACACC 660

ACATTTTACT TGTCTGCTAG GTTCGTGATA GTCGTTTAAA TTAGATACGT GAAAAAAGAT 720

TATAAATATG CAAAAGGGGA AGGGGAAGAA AAGAAAGAAA AAGGAGGGGA GAGAA ATG 778
Met
1

AAA GGC GTT AAA AAA ACC TAC GAT ATT TCT TAC ACT TTA TCC TTT TTG 826
Lys Gly Val Lys Lys Thr Tyr Asp Ile Ser Tyr Thr Leu Ser Phe Leu
            5                   10                  15

CTT GTC TTT TTC GTC TTG ATT CTA TTT CGT CCT GCC TTT TCG ATC AAC 874
Leu Val Phe Phe Val Leu Ile Leu Phe Arg Pro Ala Phe Ser Ile Asn
        20                  25                  30

ACT TTG TCG TCT ACA GAA TCT CTT ACA ATC TCA AGC AAT AGA ACA CTT 922
Thr Leu Ser Ser Thr Glu Ser Leu Thr Ile Ser Ser Asn Arg Thr Leu
    35                  40                  45

GTA TCT CCC GGT AAT GTC TTC GAG CTC GGT TTC TTC AAG ACC ACA TCA 970
Val Ser Pro Gly Asn Val Phe Glu Leu Gly Phe Phe Lys Thr Thr Ser
50                  55                  60                  65

AGT TCT CGT TGG TAT CTC GGG ATA TGG TAC AAG AAA TTT CCA TAT AGA 1018
Ser Ser Arg Trp Tyr Leu Gly Ile Trp Tyr Lys Lys Phe Pro Tyr Arg
                70                  75                  80

ACC TAT GTA TGG GTT GCC AAC AGA GAT AAC CCT CTC TCC AAT GAC ATT 1066
Thr Tyr Val Trp Val Ala Asn Arg Asp Asn Pro Leu Ser Asn Asp Ile
            85                  90                  95

GGA ACC CTT AAA ATC TCA GGC AAT AAT CTT GTC CTC CTT GAT CAC TCC 1114
Gly Thr Leu Lys Ile Ser Gly Asn Asn Leu Val Leu Leu Asp His Ser
        100                 105                 110

AAT AAA TCT GTT TGG TCG ACG AAT GTG ACT AGA GGA AAT GAG AGA TCA 1162
Asn Lys Ser Val Trp Ser Thr Asn Val Thr Arg Gly Asn Glu Arg Ser
    115                 120                 125

CCG GTG GTG GCA GAG CTT CTC GAT AAT GGA AAC TTC GTG ATG CGA GAC 1210
Pro Val Val Ala Glu Leu Leu Asp Asn Gly Asn Phe Val Met Arg Asp
130                 135                 140                 145

TCC AAT AGC AAC AAC GCA AGT CAA TTC TTG TGG CAA AGT TTC GAT TAC 1258
Ser Asn Ser Asn Asn Ala Ser Gln Phe Leu Trp Gln Ser Phe Asp Tyr
                150                 155                 160

CCT ACA GAT ACT TTG CTT CCA GAG ATG AAA CTG GGT TAC GAT CTC AAA 1306
Pro Thr Asp Thr Leu Leu Pro Glu Met Lys Leu Gly Tyr Asp Leu Lys
            165                 170                 175

ACA GGG TTG AAC AGG TTC CTT ACA TCA TGG AGA AGT TCA GAT GAT CCG 1354
Thr Gly Leu Asn Arg Phe Leu Thr Ser Trp Arg Ser Ser Asp Asp Pro
        180                 185                 190

TCA AGC GGG GAT TAC TCG TAC AAG CTC GAA CTC CGA AGA CTT CCC GAG 1402
Ser Ser Gly Asp Tyr Ser Tyr Lys Leu Glu Leu Arg Arg Leu Pro Glu
    195                 200                 205

TTT TAT CTA TCG AGT GGA TCT TTC CGA TTG CAT CGG AGT GGT CCA TGG 1450
Phe Tyr Leu Ser Ser Gly Ser Phe Arg Leu His Arg Ser Gly Pro Trp
210                 215                 220                 225

AAT GGA TTC CGA ATT AGT GGG ATA CCA GAG GAC CAA AAG TTG AGT TAC 1498
Asn Gly Phe Arg Ile Ser Gly Ile Pro Glu Asp Gln Lys Leu Ser Tyr
                230                 235                 240

ATG GTG TAC AAT TTC ACA GAG AAT AGT GAA GAG GCC GCA TAT ACA TTC 1546
Met Val Tyr Asn Phe Thr Glu Asn Ser Glu Glu Ala Ala Tyr Thr Phe
            245                 250                 255

CTA ATG ACC AAC AAC AGC TTC TAC TCG AGA TTG ACA ATA AGC TCC ACA 1594
Leu Met Thr Asn Asn Ser Phe Tyr Ser Arg Leu Thr Ile Ser Ser Thr
        260                 265                 270

GGG TAT TTT GAG CGA CTA ACG TGG GCT CCG TCA TCA GTG GTA TGG AAC 1642
Gly Tyr Phe Glu Arg Leu Thr Trp Ala Pro Ser Ser Val Val Trp Asn
    275                 280                 285

GTG TTC TGG TCT TCT CCT AAC CAC CAG TGT GAT ATG TAC AGG ATG TGT 1690
Val Phe Trp Ser Ser Pro Asn His Gln Cys Asp Met Tyr Arg Met Cys
290                 295                 300                 305

GGG CCT TAC TCT TAC TGT GAC GTG AAC ACA TCA CCG GTG TGT AAC TGT 1738
Gly Pro Tyr Ser Tyr Cys Asp Val Asn Thr Ser Pro Val Cys Asn Cys
                310                 315                 320

ATA CAA GGG TTC AGA CCC AAG AAC CGG CAG CAG TGG GAT CTG AGA ATC 1786
Ile Gln Gly Phe Arg Pro Lys Asn Arg Gln Gln Trp Asp Leu Arg Ile
            325                 330                 335

CCG ACA AGT GGG TGT ATA AGA AGG ACA CGG CTG AGC TGC AGT GGA GAT 1834
Pro Thr Ser Gly Cys Ile Arg Arg Thr Arg Leu Ser Cys Ser Gly Asp

340                 345                 350

GGT TTT ACC AGG ATG AAG AAT ATG AAG TTG CCA GAA ACT ACG ATG GCG      1882
Gly Phe Thr Arg Met Lys Asn Met Lys Leu Pro Glu Thr Thr Met Ala
    355                 360                 365

ATT GTC CAC CGA AGT ATT GGT TTG AAA GAA TGT GAG AAG AGG TGC CTT      1930
Ile Val His Arg Ser Ile Gly Leu Lys Glu Cys Glu Lys Arg Cys Leu
370                 375                 380                 385

AGC GAT TGT AAT TGT ACC GCA TTT GCA AAT GCG GAT ATC CGG AAT CGT      1978
Ser Asp Cys Asn Cys Thr Ala Phe Ala Asn Ala Asp Ile Arg Asn Arg
                390                 395                 400

GGG ACG GGT TGT GTC ATT TGG ACC GGA GAG CTT GAA GAT ATC CGG ACG      2026
Gly Thr Gly Cys Val Ile Trp Thr Gly Glu Leu Glu Asp Ile Arg Thr
            405                 410                 415

TAC TTT GCT GAC GGT CAA GAT CTT TAT GTC AGA TTG GCT GCT GCG GAC      2074
Tyr Phe Ala Asp Gly Gln Asp Leu Tyr Val Arg Leu Ala Ala Ala Asp
        420                 425                 430

CTT GTT TAGCTGTTTC TCTTAAAATA AAACACGGAT CCGACTATGA TATCGAGAAA      2130
Leu Val
    435

TCCGGATATG TAACGCAACT ATCTTAATAA GTCTACCCTA AATATATATC TGAATCAATA    2190

AATGATATAC AGATCGTATC CAAATCCTTT TTAATCCTTT TTAATATATA TAAGAAAACA    2250

TGCCATCGAA AATACAAATT TGATTATAAT ATTTTGCCGA CACCTGGAAA TTTACTTTCT    2310

TCGAATTTAT TTGTATTTGA AAGGCT                                         2336

(2) INFORMATION FOR SEQ ID NO: 2:

        (i) SEQUENCE CHARACTERISTICS:
            (A) LENGTH: 435 amino acids
            (B) TYPE: amino acid
            (D) TOPOLOGY: linear

     (ii) MOLECULE TYPE: protein

     (xi) SEQUENCE DESCRIPTION: SEQ ID NO: 2:

Met Lys Gly Val Lys Lys Thr Tyr Asp Ile Ser Tyr Thr Leu Ser Phe
  1               5                  10                  15

Leu Leu Val Phe Phe Val Leu Ile Leu Phe Arg Pro Ala Phe Ser Ile
             20                  25                  30

Asn Thr Leu Ser Ser Thr Glu Ser Leu Thr Ile Ser Ser Asn Arg Thr
         35                  40                  45

Leu Val Ser Pro Gly Asn Val Phe Glu Leu Gly Phe Phe Lys Thr Thr
    50                  55                  60

Ser Ser Ser Arg Trp Tyr Leu Gly Ile Trp Tyr Lys Lys Phe Pro Tyr
65                  70                  75                  80

Arg Thr Tyr Val Trp Val Ala Asn Arg Asp Asn Pro Leu Ser Asn Asp
                85                  90                  95

Ile Gly Thr Leu Lys Ile Ser Gly Asn Asn Leu Val Leu Leu Asp His
            100                 105                 110

Ser Asn Lys Ser Val Trp Ser Thr Asn Val Thr Arg Gly Asn Glu Arg
        115                 120                 125

Ser Pro Val Val Ala Glu Leu Leu Asp Asn Gly Asn Phe Val Met Arg
    130                 135                 140

Asp Ser Asn Ser Asn Asn Ala Ser Gln Phe Leu Trp Gln Ser Phe Asp
145                 150                 155                 160

Tyr Pro Thr Asp Thr Leu Leu Pro Glu Met Lys Leu Gly Tyr Asp Leu
                165                 170                 175

Lys Thr Gly Leu Asn Arg Phe Leu Thr Ser Trp Arg Ser Ser Asp Asp
            180                 185                 190

Pro Ser Ser Gly Asp Tyr Ser Tyr Lys Leu Glu Leu Arg Arg Leu Pro
        195                 200                 205

Glu Phe Tyr Leu Ser Ser Gly Ser Phe Arg Leu His Arg Ser Gly Pro
    210                 215                 220

Trp Asn Gly Phe Arg Ile Ser Gly Ile Pro Glu Asp Gln Lys Leu Ser
225                 230                 235                 240

Tyr Met Val Tyr Asn Phe Thr Glu Asn Ser Glu Glu Ala Ala Tyr Thr
                245                 250                 255

Phe Leu Met Thr Asn Asn Ser Phe Tyr Ser Arg Leu Thr Ile Ser Ser
            260                 265                 270

Thr Gly Tyr Phe Glu Arg Leu Thr Trp Ala Pro Ser Ser Val Val Trp
        275                 280                 285

Asn Val Phe Trp Ser Ser Pro Asn His Gln Cys Asp Met Tyr Arg Met
    290                 295                 300

Cys Gly Pro Tyr Ser Tyr Cys Asp Val Asn Thr Ser Pro Val Cys Asn
305                 310                 315                 320

Cys Ile Gln Gly Phe Arg Pro Lys Asn Arg Gln Gln Trp Asp Leu Arg
                325                 330                 335

Ile Pro Thr Ser Gly Cys Ile Arg Arg Thr Arg Leu Ser Cys Ser Gly

340                 345                 350

Asp Gly Phe Thr Arg Met Lys Asn Met Lys Leu Pro Glu Thr Thr Met
        355                 360                 365

Ala Ile Val His Arg Ser Ile Gly Leu Lys Glu Cys Glu Lys Arg Cys
    370                 375                 380

Leu Ser Asp Cys Asn Cys Thr Ala Phe Ala Asn Ala Asp Ile Arg Asn
385                 390                 395                 400

Arg Gly Thr Gly Cys Val Ile Trp Thr Gly Glu Leu Glu Asp Ile Arg
                405                 410                 415

Thr Tyr Phe Ala Asp Gly Gln Asp Leu Tyr Val Arg Leu Ala Ala Ala
            420                 425                 430

Asp Leu Val
        435

(2) INFORMATION FOR SEQ ID NO: 3:

(i) SEQUENCE CHARACTERISTICS:
(A) LENGTH: 1305 base pairs
(B) TYPE: nucleic acid
(C) STRANDEDNESS: single
(D) TOPOLOGY: linear

(ii) MOLECULE TYPE: cDNA to mRNA

(iii) HYPOTHETICAL: NO

(iii) ANTI-SENSE: NO

(vi) ORIGINAL SOURCE:
(A) ORGANISM: Brassica oleracea

(xi) SEQUENCE DESCRIPTION: SEQ ID NO: 3:

ATGAAAGGAG TAAGAAAACC CTACGATAAT TCTTATACCT TATCCTTTTT GCTTGTCTTT      60

TTCGTCTTGA TTCTATTTTG TCCTGCCTTT TCGATCAACA CTTTGTCGTC TACAGAATCT     120

CTTAGAATCT CAAGCAACAG AACACTTGTA TCTCCAGGTA ATAACTTCGA ACTCGGCTTC     180

TTCCGAACCA ACTCAAGTTC TCGTTGGTAT CTCGGGATAT GGTACAAGAA ATTGCTCGAC     240

AGAACCTATG TATGGGTTGC CAACAGAGAT AACCCACTCT CCAATGCCAT TGGAACCCTC     300

AAAATCTCAG GCAATAATCT TGTCCTCCTT GGTCACACCA ATAAATCTGT TTGGTCGACG     360

AATCTTACTA AGGAAATGAG AGACTTCCGG TGTGTGCAGA GCTTCTCTCT AATGGAAACT     420

TCGTGATGCG AGACTCCAGT AACAACGACC AAGTGAATAC TTGTGGCAAA GTTTCGATTA     480

CCCTACGGAT ACTTTGCTTC CAGAGATGAA ACTGGGTTAC GACCTCAAAA CAGGGTTGAA 540

CAGGTTCCTT ACATCATGGA GAAGTTCAGA TGATCCATCA AGCGGGGATT TCTCGTACAA 600

GCTCGAAACC CGAAGCCTTC CTGAGTTTTA TCTATGGCAT GGGATCTTTC CAATGCATCG 660

GAGTGGTCCA TGGAATGGGT CCGATTTAGT GGCATACCAG AGGACCAAAA GCTGAGTTAC 720

ATGGTGTACA ATTTCACAGA GAATAGTGAA GAGGTCGCTT ATACATTCCG AATGACCAAC 780

AACAGCATCT ACTCGAGATT GACACTAAGT TCCGAAGGGT ATTTTCAGCG ACTTACGTGG 840

AATCCGTCAA TAGGGATATG GAACAGGTTC TGGTCTTCTC CAGTGGACCC CCAGTGCGAT 900

ACATACATAA TGTGCGGGCC TTACGCTTAC TGTGGCGTGA ACACATCACC TGTTTGTAAC 960

TGTATCCAAG GGTTCAATCC CCGGAATATA CAGCAGTGGG ATCAGAGAGT CTGGGCAGGT 1020

GGGTGTATAA GGAGGACGCG GCTTAGCTGC AGTGGAGATG GTTTTACAAG GATGAAGAAC 1080

ATGAAGCTGC CAGAAACTAC GATGGCGATT GTCGACCGCA GTATTGGTGT GAAAGAATGT 1140

GAGAAGAGGT GCCTTAGCGA TTGTAATTGT ACTGCTTTTG CAAATGCGGA TATCCGGAAT 1200

GGTGGGACGG GTTGTGTGAT TTGGACCGGA CGGCTTGACG ATATGCGGAA TTACGTTGCT 1260

CACGGTCAAG ATCTTTATGT CAGATTGGCT GTTGCTGACC TTGTT 1305

(2) INFORMATION FOR SEQ ID NO: 4:

(i) SEQUENCE CHARACTERISTICS:
(A) LENGTH: 1305 base pairs
(B) TYPE: nucleic acid
(C) STRANDEDNESS: single
(D) TOPOLOGY: linear

(ii) MOLECULE TYPE: cDNA to mRNA

(iii) HYPOTHETICAL: NO

(iii) ANTI-SENSE: NO

(vi) ORIGINAL SOURCE:
(A) ORGANISM: Brassica oleracea

(xi) SEQUENCE DESCRIPTION: SEQ ID NO: 4:

ATGAAAGGCG TTAAAAAAAC CTACGATATT TCTTATACTT TATCCTTTTT GCTTGTCTTT 60

TTCGTCTTGA TTCTATTTCG TCCTGCCTTT TCGATCAACA CTTTGTCGTC TACAGAATCT 120

CTTACAATCT CAAGCAATAG AACACTTGTA TCTCCCGGTA ATGTCTTCGA GCTCGGTTTC 180

TTCAAGACCA CATCAAGTTC TCGTTGGTAT CTCGGGATAT GGTACAAGAA ATTTCCATAT 240

AGAACCTATG TATGGGTTGC CAACAGAGAT AACCCTCTCT CCAATGACAT TGGAACCCTT 300

AAAATCTCAG GCAATAATCT TGTCCTCCTT GATCACTCCA ATAAATCTGT TTGGTCGACG 360

AATGTGACTA AGGAAATGAG AGATCACCGG TGTGTGCAGA GCTTCTCGAT AATGGAAACT 420

TCGTGATGCG AGACTCCAAT AGCAACAACC AAGTCAATTC TTGTGGCAAA GTTTCGATTA 480

CCCTACAGAT ACTTTGCTTC CAGAGATGAA ACTGGGTTAC GACCTCAAAA CAGGGTTGAA 540

CAGGTTCCTT ACATCATGGA GAAGTTCAGA TGATCCGTCA AGCGGGGATT ACTCGTACAA 600

GCTCGAACTC CGAAGACTTC CCGAGTTTTA TCTATCGAGT GGATCTTTCC GATTGCATCG 660

GAGTGGTCCA TGGAATGGTT CCGAATTAGT GGGATACCAG AGGACCAAAA GTTGAGTTAC 720

ATGGTGTACA ATTTCACAGA GAATAGTGAA GAGGCCGCAT ATACATTCCT AATGACCAAC 780

AACAGCTTCT ACTCGAGATT GACAATAAGC TCCACAGGGT ATTTTGAGCG ACTAACGTGG 840

GCTCCGTCTC TAGTGGTATG GAACGTGTTC TGGTCTTCTC CTGTGAACCA CCAGTGTGAT 900

ATGTACAGGA TGTGTGGGCC TTACTCTTAC TGTGACGTGA ACACATCACC GGTGTGTAAC 960

TGTATACAAG GGTTCAGACC CAAGAACCGG CAGCAGTGGG ATCTGAGAAT CCCGACAAGT 1020

GGGTGTATAA GAAGGACACG GCTGAGCTGC AGTGGAGATG GTTTTACCAG GATGAAGAAT 1080

ATGAAGTTGC CAGAAACTAC GATGGCGATT GTCCACCGAA GTATTGGTTT GAAAGAATGT 1140

GAGAAGAGGT GCCTTAGCGA TTGTAATTGT ACCGCATTTG CAAATGCGGA TATCCGGAAT 1200

CGTGGGACGG GTTGTGTCAT TTGGACCGGA GAGCTTGAAG ATATCCGGAC GTACTTTGCT 1260

GACGGTCAAG ATCTTTATGT CAGATTGGCT GCTGCGGACC TTGTT 1305

(2) INFORMATION FOR SEQ ID NO: 5:

(i) SEQUENCE CHARACTERISTICS:
(A) LENGTH: 1307 base pairs
(B) TYPE: nucleic acid
(C) STRANDEDNESS: single
(D) TOPOLOGY: linear

(ii) MOLECULE TYPE: cDNA to mRNA

(iii) HYPOTHETICAL: NO

(iii) ANTI-SENSE: NO

(vi) ORIGINAL SOURCE:
(A) ORGANISM: Brassica oleracea

(xi) SEQUENCE DESCRIPTION: SEQ ID NO: 5:

ATGAAAGGAG TAAGAAAAAA CTACAATAAT TCTTACATCT TATCCTTTTT GCTTGTCTTT 60

TTCGTCTCGA CCCTATTTTG TCATGCCTTT TCGATCAACA CTTTGTCGTC TATAGAATCT 120

CTTACAATCT CCAGCAACAG AACACTTGTA TCTCCCGGTA ATGTCTTCGA GCTCGGCTTC 180

TTCAGAACCA ACTCAAGTTC TCGTTGGTAT CTCGGGATAT GGTACAAGAA AGTGTCCGAC 240

AGAACCTATG TATGGGTTGC CAACAGAGAT AACCCTCTCT CCAGTTCCAT TGGAACCCTC 300

AAAATCTCTG GCAATAATCT TGTCATCCTT GATCACTCTA ATAAATCTGT TTGGTCGACG 360

AATCTAACTA AGGAAATGAG AGATCTCCGG TGTGTGCAGA CGTTCTCGCT AATGGAAACT 420

TCGTGATGCG AGACTCCAAT AACAACGACC AAGTGGATTC TTGTGGCAAA GTTTCGATTT 480

CCCTACAGAT ACTTTGCTTC CAGAGATGAA ACTGAGTTAC GACCTCAAAA CAGGGCTGAA 540

CCGGTTCCTT ACATCACGGA GAAGTTCAGA TGATCCGTCA AGCGGAGATT TCTCATACAA 600

GCTCGAACCT CGAAGACTTC CTGAATTTTA TCTATCGAGT GGGGTCTTTC TATTGTATCG 660

GAGTGGTCCA TGGAATGGAT CCGATTTAGT GGCCTACCAG ATGACCAAAA GTTGAGTTAC 720

TTGGTGTACA TTTCACTGGA ATGAGAGTCG CTTATAAATT CCGAATGACC AACAACAGCT 780

TCTACTCGAG ATTATCTGTA AGTTTCTCAG GGTATATTGA GCAACAGACG TGGAATCCGT 840

CTCTAGGGAT GTGGAACAGT TTCTGGGCTT TTCCGTTGGA TTCACAGTGC TATACGTACA 900

GAGCGTGTGG GCCTTACTCT TACTGTGTCG TGAACACATC AGCGATTTGT AACTGTATCC 960

AAGGGTTCAA TCCCTCGAAT GTGCAGCAGT GGGATCAGAG AGTCTGGGCA GGTGGGTGTA 1020

TAAGGAGGAC GCGGCTTAGC GGCAGTGGAG ATGGCTTTAC AAGGATGAAG AATATGAAGC 1080

TGCCAGAAAC TACGATGGCG ATTGTCGACC GCAGTATTGG TGTGAAAGAA TGTGAGAAGA 1140

GGTGCCTTAA CGACTGTAAT TGTACCGCGT TTGCAAATGC GGATATCCGG AATGGTGGGA 1200

CGGGTTGTGT GATTTGGACT GGAGAGCTCG AGGATATGCG GAGTTACGCT GATGGCGCTA 1260

CTGACAGTCA AGATCTTTAT GTCAGATTGG CTGCAGCTGA CATTGTT 1307

(2) INFORMATION FOR SEQ ID NO: 6:

(i) SEQUENCE CHARACTERISTICS:
(A) LENGTH: 1308 base pairs
(B) TYPE: nucleic acid
(C) STRANDEDNESS: single
(D) TOPOLOGY: linear

(ii) MOLECULE TYPE: cDNA to mRNA

(iii) HYPOTHETICAL: NO

(iii) ANTI-SENSE: NO

(vi) ORIGINAL SOURCE:
(A) ORGANISM: Brassica oleracea

(xi) SEQUENCE DESCRIPTION: SEQ ID NO: 6:

ATGAAAGGCG TAAGAAAAAC CTACGACAGT TCCTGCACAT TATCCTTTTC GCTTGTCTTT 60

TTCGTTATGA TTATATTTCA CCCCGTCTTT TCGATCAACA CTTTGTCGGC TACAGAATCT 120

CTTACGATCT CAAGCAACAG AACACTTGTA TCTCCCGGTA ATGTCTTCGA ACTCGGTTTC 180

TTCAGAACCA CCTCGAGTTC TCGTTGGTAT CTCGGGATAT GGTACAAGAA ATTGTCCAAC 240

AGAACCTATG TATGGGTTGC CAACAGAGAT AACCCTCTTT CCAACTCCAC TGGAACCCTC 300

AAAATCACAA GCAATAATCT TGTTATCCTT GGTCACTCCA ATAAATCTAT TTGGTCCACG 360

AATCGTACTA GGGAAATGAG AGGTCTCCGG TGTGTGCAGA GCTTCTCGCT AATGGAAACT 420

TCTTGATGCG AGACTCCAAT AACAACCGCT AAGTCGATTC TTGTGGCAAA GTTTCGATTA 480

CCCTACAGAT ACTTTGCTTC CAGAGATGAA GCTGGGTTAC GACCTCAAAA CAGGGCTGAA 540

CAGATTCCTT ACATCATGGA GAAGTTCTGA TGATCCATCA AGCGGGGATT TCTCATACAA 600

GCTCAGAGCC CGAAGGCTTC CTGAACTTTA TCTATCGAGT GGGATCTTTC GAGTGCATCG 660

GATCGGTCCA TGGAACGGAT CCGATTTAGT GGCATACCAG ATGACCGAAA GCTGAGTTAC 720

TTGGTGTACA ATTTCACAGA GAATAATGAG GAGGTCGCTT ATACATTCCG AATGACCAAC 780

AACACCATCT ACTCGAGATT GACAGTAAGT TTCTCAGGGT ATATTGAGCG ACAGACGTGG 840

AATCCGTCTA TAGGGATGTG GAACGTGTTC TGGTCTTTTC CATTGGACTC ACAGTGCGAT 900

GCGTACAGAG CGTGTGGGCC TTACTCTTAC TGTGACGTGA ACACATCACC GATTTGTAAC 960

TGTATCCAAG GGTTCAATCC CTCGAATGTG GAGCAGTGGG ATCAGAGAGT CTGGGCAAAT 1020

GGGTGTATAA GGAGGACGCG ACTTAGCTGC AGTGGAGATC GTTTTACCAT GATGAAGAAC 1080

ATGAAGTTGC CAGAAACTAC GATGGCGTTT GTCGACAGGA GTATTGGTGT GAAAGAATGT 1140

GAGAAGAGGT GCCTTAGCGA TTGTAATTGC ACCGCGTTTG CAAATGCGGA TATCCGGAAT 1200

GGTGGGGCGG GTTGTGTGAT TTGGACCGGA CGGCTTGACG ATATGCGGAA TTACGCTGCT 1260

GATCACGGTC AAGATCTTTA TGTCAGATTG GCTGCTGCTA ACCTTGTT 1308

(2) INFORMATION FOR SEQ ID NO: 7:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iii) ANTI-SENSE: NO

(vi) ORIGINAL SOURCE:
(B) STRAIN: Oligonucleotide

(xi) SEQUENCE DESCRIPTION: SEQ ID NO: 7:

GAAAAAGGAG GGGATCCAAA TGAAAGGCG 29

## Description

The present invention relates to tissue specific promoter sequences that are capable of directing the expression of an associated structural gene to the reproductive tissue of a plant, and to a method of obtaining reproductive tissue specific expression of a gene Plant science has recognized for many years that hybridization of closely related plants often results in the production of an offspring (F₁) generation having a combination of desirable traits which previously were possessed separately by the parent plants. Also recognized is that hybrid plants of various crop species commonly have possessed vigor or heterosis which has contributed significantly to the species' crop yield. Accordingly, many hybrid crosses have been of considerable economic importance.

Since plants selected for hybridization studies are commonly capable of undergoing both self-pollination (i.e., the plants are self compatible) and cross-pollination, the desired hybrid crosses have been difficult to achieve on a reliable basis while operating on a commercial basis. In order to achieve hybrid crosses on a commercial scale, it is necessary to have controlled cross-pollination in the substantial absence of self-pollination (i.e., plants that are self-incompatible).

In more than 3,000 species representing diverse angiosperm families, self-fertilization is blocked by a genetically controlled mechanism termed "self-incompatibility". This phenomenon facilitates out-crossing by inhibiting growth of self pollen in pistil tissue. Following self-pollination, interaction between the stigma or style and pollen elicits a defined morphological response preventing normal pollen tube growth. Fertilization is thus prevented as the pollen tubes are unable to penetrate the full length of the stigma and style and the gamete nuclei of the pollen are unable to reach the female gametophyte tissue. The specificity of this interaction is determined by one or more genetic loci, and requires the identity of alleles carried by the male and female parents.

The control of incompatibility is attributed to a single genetic locus with multiple alleles. As with other allelomorphs, any two alleles may be carried by a diploid plant, and incompatibility occurs if the alleles expressed in pollen and pistil are identical.

Incompatibility systems are generally divided into two major groups: the gametophytic system in which the phenotype of a pollen grain is determined by the haploid pollen genotype is the first, and the sporophytic system in which the pollen phenotype is determined by the genotype of the parental plant is the second. In the sporophytic system of incompatibility, exemplified by the genus Brassica, and other crucifers belonging to the family Brassicaceae (which includes such plants as broccoli, cabbage, kale, mustards, oil seed crops and radish), the self-incompatible inhibition is localized at the stigma surface and occurs within minutes by the failure of self-pollen to germinate and/or invade the papillar cells on the outer surface of the stigma. Thus, self-fertilization is prevented.
In this Brassicaceae family, self-incompatibility is under the control of a single multi-allelic locus (numerous alleles have been identified in natural populations), called the S-locus, and the phenotype of pollen is determined by the diploid genotype of the sporophyte [see A.J.Bateman (1955)]. While the mechanism of pollen recognition in plants is not understood, the observation that a self-incompatibility response occurs when the same S-allele is active in pollen and stigma suggests that pollen inhibition is based on the expression of a single S-locus gene in the male and female structures of the flower. Furthermore, the sporophytic control of pollen phenotype has been postulated to result from the expression of this S-locus gene in the tapetum, the layer of sporophytic cells that lines the anther and whose function is to nourish the developing pollen grains [see J.Heslop-Harrison (1968) and J.Heslop-Harrison (1975)]. Based largely on ultrastructural observations, this layer of cells is thought to act as nurse cells for developing microspores, and to synthesize and release materials that are incorporated into the outer coat of pollen grains, the exine, just prior to and during tapetal dissolution late in pollen development.

A gene involved in the specific recognition of self pollen is also expected to be sporophytically expressed in the host's anthers during pollen development. While blot analysis of anther RNA has identified a low-abundance transcript with homology to the S-locus glycoprotein gene [see J.B.Nasrallah et al (1989)] these studies could not distinguish between expression of the S-locus glycoprotein related genes found in the Brassica genome [see K.G.Dwyer et al (1989)]. The putative S-locus genetic protein of pollen has and continues to remained elusive.

Perhaps the most experimentally tractable route towards a molecular analysis of the genetic control of incompatibility is suggested by the detection of antigens specific to various S-locus alleles in stigma homogenates from different Brassica strains. These antigens have been shown to correspond to glycoproteins which may be resolved in various electrophoretic systems. Several lines of evidence suggest that these glycoproteins play an important role in incompatibility: (1) the mobilities of these molecules vary in stigma extracts derived from Brassica strains with different S-locus alleles; (2) these molecules are found exclusively in the stigma (specifically in the surface papillar cells on the stigma surface), the site of the initial contact between the pollen and stigma; (3) an increased rate of synthesis of these S-locus specific glycoproteins in the developing stigma correlates well with the onset of the incompatibility reaction in the stigma; and (4) in the stigma of self-incompatible lines of B. oleracea (kale), B. campestris, and the closely related Raphanus sativus (radish) glycoproteins have been identified which cosegregate with their corresponding S-alleles in the F₁ and F₂ generations, indicating that the gene responsible for this polymorphism is at the S-locus.

These glycoproteins have been designated S-locus specific glycoprotein ("SLSG"). Each S genotype has its own unique glycoprotein, and to date more than twenty different SLSG have been correlated with S-alleles. Furthermore, SLSG encoded by different S alleles exhibit extensive polymorphism and are distinguishable from one another on the basis of their characteristic charge, molecular weight and antigenic properties. In addition, SLSG accumulate during stigma development and are synthesized at a maximal rate coincident with the acquisition by the developing stigma of the ability to reject self pollen.

The cell-specific expression of these genes in the stigma has been well characterized. S-locus gene transcripts were localized by in situ hybridization to the papillar cells of the stigma surface [see J.B.Nasrallah et al (1988)], and S-locus gene glycoprotein products were shown by ultrastructural immunocytochemistry to accumulate in the papillar cell walls [see M.K.Kandasamy et al (1989)]. These results, combined with the genetic linkage of S-locus glycoprotein genes to the S-locus, have led to the finding , that these genes encode the stigmatic determinants of self-incompatibility.

Finally analysis of Brassica genomic DNA has revealed extensive restriction fragment length polymorphisms which cosegregate with the corresponding S alleles in genetic crosses. These developmental, cytological and genetic data strongly suggest that SLSG are the products of the S-locus and that they act as recognition substances in the self-incompatibility reaction.

When used as a probe on blots of genomic DNA prepared from Brassica, Raphanus, Arabidopsis and other crucifers, SLSG-encoding cDNAs hybridize with several restriction fragments indicating that SLSG-related sequences occur as a multigene family in crucifers.

Members of this multigene family can be grouped into two classes. One class comprises genes linked at the S-locus including 1) the SLSG structural gene, referred to as the SLG gene, and 2) additional related genes. Another class comprises genes related to SLG genes but unlinked to the S-locus, and are designated "S-locus related" (SLR) genes. All these genes also exhibit considerable (60-80 %) homology at the amino acid level.

Within any one S-allele homozygote, the SLG gene can be identified from among the other related gene copies by hybridization to a gene-specific probe derived from the 3 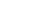 untranslated end of SLSG-encoding cDNA. By using this 3'-end gene specific probe, the SLG gene can be isolated from the S13, S14 and S22 homozygous lines of Brassica oleracea, and from the S8 homozygous line of B. campestris. The cloned genes are referred to as SLG-13, SLG-14, SLG-22 and SLG-8 respectively. The restriction maps of these genes and flanking regions are shown in Figure 2.

The determination of a substantially pure DNA base sequence coding for an S-locus specific glycoprotein from a self-incompatible strain of Brassica, the construction of novel recombinant DNA transfer vectors containing the cloned DNA, and transformed microorganisms transformed by the recombinant DNA transfer vectors have been already described in EP application 212,385.

SLR genes have been isolated from a number of different S-allele homozygous genotypes. To date, two SLR genes have been shown to be expressed. These genes are referred to as SLR1 and SLR2. Both genes are expressed in the papillar cells of the stigma surface and have the same pattern of expression during flower development as the SLG genes. They therefore have common regulatory sequences that direct their cell-type specific expression.

The SLR1 gene can be isolated from the S2, S6, S13, S15, S22 homozygotes of B. oleracea and from the Yellow Sarson variety of B. campestris [except for S15, these have been reported in Lalonde et al (1989)].

The SLR2 gene can be isolated from the S2, S5, S6 and S15 homozygous genotypes of B.oleracea. A map of SLR2-cDNA isolated from the S2 and S15 genotypes is shown in Figure 3.

Also comprised by the present invention is a method of obtaining tissue specific expression of a gene in a plant comprising:
(a) isolating a tissue specific plant promoter from the reproductive tissue of a donor plant;
(b) linking the tissue specific plant promoter with a structural gene, preferably a structural gene encoding for a S-locus specific glycoprotein, such that the structural gene is specifically expressed only in the reproductive tissue, preferably in the pistil and/or anther of a recipient plant.

Preferred is a method for obtaining a male and/or female sterile plant which method comprises the following steps:
(a) isolating a tissue specific plant promoter from the reproductive tissue of a donor plant;
(b) operably linking the tissue specific plant promoter with a DNA sequence that encodes for a toxin protein;
(c) inserting the resulting chimeric gene into the genome of a recipient plant;
(d) allowing the recipient plant to mature and assaying the flower parts, especially the pistil and/or the anther, of the mature plant for the expression of the toxin gene; and
(e) selecting those plants which show expression of the toxin gene.

A further embodiment of the present invention is a recombinant DNA molecule that comprises a chimeric genetic construct comprising a toxin gene. Within the scope of the present invention the term 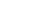 toxin gene 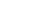 is to be understood as comprising such genes that encode for a protein or polypeptide that is capable of conferring a male and/or female sterility to the target plant, selected from the group consisting of nuclease genes such as RNAse or DNAse genes, proteolytic enzyme genes, lectin genes, protein synthesis inhibitor genes, genes that control cell division, lytic peptide genes such as, for example, the thionin gene, the cecropin gene, the maganin gene or the defensin gene, lipase genes, ribozyme genes, antisense genes, phospholipase genes, genes for enzymes in the pathway for synthesis of a phytohormone and genes for an inhibitor of cell wall synthesis.

The present invention further relates to a recombinant DNA molecule that comprises a chimeric genetic construct that comprises one or more further structural genes, preferably a reporter gene the expression of which in a transgenic cell is indicative of the incorporation of the associated structural gene into the transgenic cell's genome and most preferably a reporter gene that is capable of imparting herbicide tolerance. Especially preferred is a gene that is capable of imparting herbicide tolerance to one or more herbicides selected from the group consisting of hygromycin, kanamycin, phosphinotricin, asulam, sulfonylureas, glyphosate, bromoxymil and imidazolinones, wherein the second structural gene is preferably genetically linked with the structural gene. A further embodiment of the present invention is a recombinant DNA vector, and especially a recombinant DNA transfer vector comprising:
(a) a structural gene, and
(b) a transformation vector containing DNA sequences capable of driving the expression of said structural gene when the construct is incorporated into a plant.

The present invention further relates to a recombinant DNA transfer vector that comprises one or more further structural genes, preferably a reporter gene the expression of which in a transgenic cell is indicative of the incorporation of the associated structural gene into the transgenic cell's genome and most preferably a reporter gene capable of imparting herbicide tolerance, especially herbicide tolerance to one or more herbicides selected from the group consisting of hygromycin, kanamycin, phosphinotricin, asulam, sulfonylureas, glyphosate, bromoxymil and imidazolinones, wherein the gene for herbicide tolerance is genetically linked with the structural gene.

Also preferred is a recombinant DNA transfer construct comprising the fusion of:
(a) a toxin gene and
(b) a reporter gene the expression of which in a transgenic cell is indicative of the incorporation of the structural gene into the transgenic cell's genome.

Further preferred within the present invention is a recombinant DNA transfer construct wherein the structural gene is operably linked with a tissue specific plant promoter such that the structural gene is specifically expressed only in the reproductive tissue, preferably in the pistil and/or anther of a recipient plant.

Also comprised are female and/or male sterile plants and the asexually derived progeny thereof that still contains the trait of male and/or female sterility, comprising a structural gene which encodes for the production of a toxin gene, operably linked to a plant specific promoter that is capable of directing the expression of the associated toxin gene in the reproductive tissue, preferably in the anther and/or pistil, of a recipient plant.

A further embodiment of the present invention is a tissue specific plant promoter comprising a DNA sequence comprising a region of an S-locus specific gene that directs the expression of a linked structural gene in the reproductive tissue, preferably in the anther and/or pistil, of a recipient plant.

A more complete understanding of the present invention may be had by reference to the genetic sequence for the SLG gene allele designated as S13 and isolated from Brassica oleracea, shown in SEQ ID NO:1 . In this sequence, the amino acid translation is also provided for the region of 776 to 2080 (SEQ ID NO: 2).

The SLG gene can be differentiated from among other S-related copies by specific hybridization to a probe derived from the 3 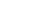 end of SLSG-encoding cDNA. Using this 3 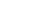 gene specific probe (the region 2107 to 2216 in the S13 sequence), it has been possible to isolate SLG genes from a number of different S-allele homozygous genotypes.

In general, the sequences responsible for the correct tissue and cell type specific and developmental regulation of the SLG genes have been shown to reside in sequences that lie 5 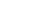 to the transcribed and protein-coding region. Primer-extension experiments have shown that the 5 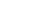 -end of the mRNA transcript of the SLG-13 gene lies 15 base pairs 5 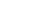 to the initiating methionine codon. In addition, transformation experiments have shown that an SLG-13 gene containing only 411 base pairs 5 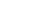 to the initiating methionine codon is expressed specifically in the pistil of transgenic Nicotiana in manner identical to that described for the gene construct containing 4.2 kilobases of 5 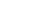 sequences. Therefore the promoter sequence is contained in the region extending from position -17 to position - 411, more particularly in the region extending from approximately -140 to -411 [position +1 being the A of the initiating ATG].

In addition to the SLG-13 gene sequence given in SEQ ID NO: 1, the cDNA sequences of S6, S13, S14 and S22 are given in SEQ ID Nos: 3 to 6.

The sequence alignments and differences in the coding region among these four S-locus alleles of Brassica oleracea are given in Figure 5 . The nucleotide sequences were determined from the cDNA inserts of lambda gt10 clones isolated from stigma cDNA libraries. The alignments are further based upon predicted amino acid sequence and nucleotide sequence similarity, attempting to minimize both differences and gaps. Any gaps in the sequence are indicated by dots in the sequence.

To determine if the S-locus glycoprotein gene is expressed in anthers, and to define the site and timing of this expression, the present invention utilizes an E.coli b-glucuronidase (GUS) reporter gene fusion to monitor S-locus glycoprotein gene promoter activity in tissues of developing flowers. It goes without saying that within the present invention any other suitable reporter gene can also be used for that purpose. A chimeric gene is constructed by inserting a 3.65kb BamHI fragment containing the 5 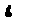 upstream region of the S-locus glycoprotein gene, especially a SLG₁₃ gene (although other SLG genes described herein may be used as well) derived from Brassica oleracea [see J.B.Nasrallah (1988)] (the 3.65kb BamHI promoter fragment extends from a BamHI site 3,650kb upstream of the initiating ATG codon in the S-locus gene isolated from the S₁₃ homozygote to a BamHI site introduced 8bp upstream of the ATG codon by site-directed mutagenesis) into the GUS expression vector pBI101 [see R.A.Jefferson et al (1987)]. The resulting vector plasmid, pMKT8 is introduced into Arabidopsis thaliana, a member of the Brassicaceae that has a short generation time, is easily transformed, and in which the self incompatibility trait is not known to occur. However, the rapid flowering of regenerated transgenic plants makes Arabidopsis well suited for the analysis of flower-specific promoters such as those of the S-locus glycoprotein gene. Root segments are inoculated with Agrobacterium cells harboring pMKT8, and transgenic plants are selected based on kanamycin resistance [transgenic plants were produced following infection of Arabidopsis thaliana (L.) Heynh. strain C24 root explants with Agrobacterium tumefaciens as described in PNAS USA 85:5536 (1988); the Agrobacterium strain is pCIB542/A136, a strain derived from helper plasmid pEHA101 containing binary vector pMKT8; transformants are selected, and seeds produced by these plants are germinated on medium containing kanamycin in a suitable concentration].

In addition to the reporter gene fusion described above, a second fusion gene construct is made in order to further characterize the sites of S-gene expression as well as to study the function of tissues that express the S-gene. In this construct, the 3.65kb S₁₃ gene promoter fragment is fused to the coding sequence of the Diphtheria toxin A polypeptide in the plant transformation vector pBIN19 [see M Bevan, Nucleic Acids Res. 12:8711 (1984)] to generate plasmid pMKT17. Diphtheria toxin A polypeptide is known to block protein synthesis through ADP-ribosylation of elongation factor II in many organisms, including plants [see R.J. Collier (1977)], and has also been demonstrated to have nuclease activity [see M.P. Chang et al (1989)]. These cytotoxic effects allow a single molecule of Diphtheria toxin A polypeptide to cause cell death [see M.Yamizumi et al (1978)], and so Diphtheria toxin A polypeptide can serve as a very sensitive indicator of gene expression. Gene fusions to Diphtheria toxin A polypeptide have been used to genetically direct cell death in a variety of mammalian tissues [see R.R. Behringer et al (1988)].

The S₁₃: Diphtheria toxin A polypeptide toxic gene fusion can be introduced into tobacco by Agrobacterium-mediated transformation.

In order to ensure the expression of a structural gene in the plant cell, it is advantageous for the coding gene sequence first to be linked in operable manner to expression sequences capable of functioning in plant cells.
The hybrid gene constructions within the scope of the present invention therefore contain in addition to one or more structural gene(s) also expression signals which include both promoter and terminator sequences and other regulatory sequences of the 3 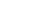 and 5 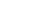 untranslated regions.

Any promoter and any terminator capable of bringing about an induction of the expression of a coding DNA sequence (structural gene) may be used as a constituent of the hybrid gene sequence. Especially suitable are expression signals originating from genes of plants or plant viruses. Examples of suitable promoters and terminators are those of the Cauliflower Mosaic Virus genes (CaMV) such as the 35S and 19S expression signals or homologous DNA sequences that still have the characteristic properties of the mentioned expression signals. Also suitable are bacterial expression signals, especially the expression signals of the nopaline synthase genes (nos) or the octopine synthase genes (ocs) from the Ti-plasmids of Agrobacterium tumefaciens.

In accordance with the invention there may be used as starting material for the 35S transcription control sequences, for example, the ScaI fragment of the CaMV strain "S", which includes the nucleotides 6808-7632 of the gene map [Frank G et al (1980)].

The 19S promoter and 5 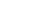 untranslated region is located on a genome fragment between the PstI site (position 5386) and the HindIII site (position 5850) of the CaMV gene map [Hohn et al (1982)]. The corresponding terminator and 3 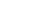 untranslated region is located on an EcoRV/BgIII fragment between positions 7342 and 7643 of the CaMV genome.

Also suitable within the scope of this invention are the expression signals of the CaMV strain CM 1841, the complete nucleotide sequence of which is described in Gardner RC et al (1981).

A further effective representative of a plant promoter that may be used is an over-producing plant promoter. Provided that this type of promoter is operably linked to the gene sequence that codes for a desired gene product, it should be capable of mediating the expression of the said gene sequence.

Over-producing plant promoters that may be used within the scope of the present invention include the promoter of the small subunit (ss) of ribulose-1,5-bisphosphate carboxylase from soybeans and also the promoter of the chlorophyll-a/b-binding protein. These two promoters are known for the fact that they are induced by light in eukaryotic plant cells [see, for example, Genetic Engineering of Plants, An Agricultural Perspective, Cashmore A (1983)].

Preferred within the present invention are tissue specific plant promoters that direct the expression of a linked structural gene in the reproductive tissue, preferably in the anther and/or pistil, of a recipient plant. Especially preferred is a tissue specific plant promoter comprising a DNA sequence comprising a region of an S-locus specific gene, preferably a region comprising a 3.65 kb BamHI-fragment of the SLG-13 gene, more preferably a region of the SLG-13 gene extending from approximately position -17 to approximately position - 411, and most preferably a region of the SLG-13 gene extending from approximately position -150 to approximately position -411 of the SLG-13 gene, which region is capable of directing the expression of an associate
d structural gene in the reproductive tissue, preferably in the anther and/or pistil, of a recipient plant.

Besides the promoter region there are further regulatory DNA sequences known that may also he used for the construction of chimeric gene constructs including those sequences, for example, that are capable of regulating the transcription of an associated DNA sequence in plant tissues in the sense of induction or repression.

There are, for example, individual plant genes that are known to be induced by various internal and external factors, such as plant hormones, heat shock, chemicals, pathogens, oxygen deficiency, light, etc..

As an example of gene regulation by a plant hormone, mention should here be made of abscisic acid (ABS), which is known to induce the excess of mRNAs that occurs during the late embryonal phase in cotton. A further example is gibberellic acid (GA3) which induces malate synthase transcripts in castor beans and isoenzymes of a-amylase in the aleurone layers of barley.

The activity of glucanase and chitinase in bean leaves can be markedly increased by treatment with the stress hormone ethylene. In the case of chitinase, this induction effect is controlled via the promoter of the chitinase gene, and it was possible to demonstrate this by reporter gene tests using a promoter from the chitinase gene of beans (Phaseolus vulgaris).

The regulation of heat-shock-sensitive protein genes of soybeans has been studied in detail. Treating the plants for several hours at a temperature of 40°C results in the de novo synthesis of so-called heat-shock proteins. A large number of those genes have since been isolated, and their regulation has been analyzed in detail. The expression of those genes is controlled primarily at the transcription level. For example, if the promoter of the hps70 gene is fused with the neomycin phosphotransferase II (NPT II) gene, the chimaeric gene so formed can be induced by a heat shock (Spena et al, 1985).

Another class of genes that are inducible in plants comprises the light-regulated genes, especially the nuclear-coded gene of the small subunit of ribulose-1,5-bisphosphate carboxylase (RUBISCO). Morelli et al (1985) have shown that the 5'-flanking sequence of a RUBISCO gene from the pea is capable of transferring light-inducibility to a reporter gene, provided the latter is linked in chimaeric form to that sequence. It has also been possible to extend this observation to other light-induced genes, for example the chlorophyll-a/b-binding protein.

The alcohol dehydrogenase genes (adh genes) of maize have been the subject of intensive research. The adh1-s gene from maize was isolated, and it was shown that a part of the 5'-flanking DNA is capable of inducing the expression of a chimaeric reporter gene (e.g. chloramphenicol acetyl transferase; CAT) when the temporarily transformed tissue was subjected to anaerobic conditions [Howard et al (1987)].

A further group of regulatable DNA sequences comprises chemically regulatable sequences that are present, for example, in the PR (pathogenesis-related) protein genes of tobacco and are inducible by means of chemical regulators.

The regulatable DNA sequences mentioned by way of example above may be of both natural and synthetic origin, or they may comprise a mixture of natural and synthetic DNA sequences.

The present invention therefore also includes chimaeric genetic constructions that may contain, in addition to the promoter sequences in operable linkage with a structural gene, further regulatory sections of DNA sequence permitting, for example, specifically controlled induction or repression of gene expression.

The various sections of sequence can be linked to one another by methods known per se to form a complete DNA sequence expressible in plant cells. Suitable methods include, for example, the in vivo recombination of DNA sequences having homologous sections and the in vitro linking of restriction fragments.

As cloning vectors there are generally used plasmid or virus (bacteriophage) vectors having replication and control sequences originating from species that are compatible with the host cell.

The cloning vector generally carries an origin of replication and, in addition, specific genes that lead to phenotypic selection features in the transformed host cell, especially to resistance to antibiotics or to specific herbicides. The transformed vectors can be selected on the basis of those phenotypic markers after transformation in a host cell.

Selectable phenotypic markers that may be used within the scope of this invention include, for example, resistance to ampicillin, tetracycline, hygromycin, kanamycin, methotrexate, G418 and neomycin, but this list, which is given only by way of example, is not intended to limit the subject of the invention.

Suitable host cells within the scope of this invention are prokaryotes, including bacterial hosts, for example A. tumefaciens, E. coli, S. typhimurium and Serratia marcescens, and also cyanobacteria. Eukaryotic hosts, such as yeasts, mycelium-forming fungi and plant cells, may also be used within the scope of this invention.

The splicing of the hybrid gene construction according to the invention into a suitable cloning vector is carried out using standard methods, such as those described in Maniatis et al (1982).

As a rule, the vector and the DNA sequence to be spliced in are first cleaved with suitable restriction enzymes. Suitable restriction enzymes are, for example, those that yield fragments having blunt ends, for example SmaI, HpaI and EcoRV, or enzymes that form cohesive ends, for example EcoRI, SacI and BamHI.

Both fragments having blunt ends and those having cohesive ends that are complementary to one another can be linked again using suitable DNA ligases to form a continuous uniform DNA molecule.

Blunt ends can also be produced by treatment of DNA fragments that have projecting cohesive ends with the Klenow fragment of the E. coli DNA polymerase by filling the gaps with the corresponding complementary nucleotides.

On the other hand, cohesive ends can also be produced by artificial means, for example by the addition of complementary homopolymeric tails to the ends of a desired DNA sequence and of the cleaved vector molecule using a terminal deoxynucleotidyl transferase, or by the addition of synthetic oligonucleotide sequences (linkers) that carry a restriction cleavage site, and subsequent cleavage with the appropriate enzyme.

The cloning vector and the host cell transformed by that vector are generally used to increase the number of copies of the vector. With an increased number of copies it is possible to isolate the vector carrying the hybrid gene construction and prepare it, for example, for insertion of the chimaeric gene sequence into the plant cell.

In a further process step, these plasmids or derivatives thereof may be used as transfer vectors to insert the structural genes coding for a desired gene product or non-coding DNA sequences having a regulatory function, for example anti-sense DNA, into the plant cell and, optionally, to integrate them into the plant genome.

Preferred within the scope of the present invention is a recombinant DNA transfer vector comprising:
(a) a toxin gene, and
(b) a transformation vector containing DNA capable of driving the expression of said structural gene when the construct is incorporated into a plant.

Also preferred is a recombinant DNA transfer vector that further comprises a gene capable of imparting herbicide tolerance, preferably a gene capable of imparting herbicide tolerance to one or more herbicides selected from the group consisting of hygromycin, kanamycin, phosphinotricin, asulam, sulfonylureas, glyphosate, bromoxymil and imidazolinones, wherein the gene for herbicide tolerance is genetically linked with the structural gene coding for the S-locus specific glycoprotein.

Further preferred within the present invention is a recombinant DNA transfer construct wherein the structural gene is operably linked with a tissue specific plant promoter such that the structural gene is specifically expressed only in the reproductive tissue, preferably in the pistil and/or anther of a recipient plant.

The above described transfer vectors comprising the chimeric genetic constructions may be introduced into the target plant by a variety of processes which have come into existence for introducing DNA into plant cells.

One possible method consists, for example, in bringing plant cells into contact with viruses or with Agrobacterium. This may be achieved by infecting sensitive plant cells or by co-cultivating protoplasts derived from plant cells. Within the scope of this invention, Cauliflower Mosaic Virus (CaMV) may also be used as a vector for the insertion of the chimaeric genetic construction according to the invention into the plant. The total viral DNA genome of CaMV is integrated into a bacterial parental plasmid to form a recombinant DNA molecule that can be propagated in bacteria. After cloning has been carried out, the recombinant plasmid is cleaved, using restriction enzymes, either randomly or at very specific, non-essential sites within the viral part of the recombinant plasmid, e.g. within the gene that codes for the transferability of the virus by aphids, and the hybrid gene construction is cloned into that cleavage site.

A small oligonucleotide, a so-called linker, which has a single, specific restriction recognition site, may also be integrated. The recombinant plasmid so modified is cloned again and further modified by splicing the hybrid gene construction into a restriction site that occurs only once.

The modified viral portion of the recombinant plasmid can then be cut out of the bacterial parental plasmid and used for the inoculation of the plant cells or of the whole plants.

Another method of inserting the chimaeric gene construction into the cell makes use of the infection of the plant cell with Agrobacterium tumefaciens and/or Agrobacterium rhizogenes, which has been transformed using the said gene construction. The transgenic plant cells are then cultured under suitable culture conditions known to the person skilled in the art, so that they form shoots and roots and whole plants are finally formed.

A further possible method of transforming plant material comprises mixed infection using both Agrobacterium rhizogenes and transformed Agrobacterium tumefaciens, as described by Petit et al (1986) for the transformation of carrots. The mixing ratio must be such that the root colonies formed on the basis of the A. rhizogenes transformation also contain a high proportion of A. tumefaciens Ti-plasmids. This may be achieved, for example, by applying A. rhizogenes and A. tumefaciens together to the plant material in known manner in a mixing ratio of from 1:1 to 1:100, preferably in a mixing ratio of 1:10. The transgenic plants are then cultured under suitable culture conditions known to the person skilled in the art, so that they form shoots and roots and whole plants are finally formed. The two Agrobacterium species are advantageously not mixed until shortly before the actual inoculation of the plant material to be transformed.

The chimaeric gene construction according to the invention can therefore be transferred into suitable plant cells by means of, for example, the Ti-plasmid of Agrobacterium tumefaciens or the Ri-plasmid of Agrobacterium rhizogenes. The Ti-plasmid or Ri-plasmid is transferred to the plant in the course of infection by Agrobacterium and integrated in stable manner into the plant genome.

Both Ti-plasmids and Ri-plasmids have two regions that are essential for the production of transformed cells. One of those regions, the transfer-DNA (T-DNA) region, is transferred to the plant and leads to the induction of tumors. The other region, the virulence-imparting (vir) region, is essential only for the formation of the tumors, not for their maintenance. The dimensions of the transfer-DNA region can be enlarged by incorporation of the chimaeric gene construction without the transferability being impaired. By removing the tumor-inducing genes and incorporating a selectable marker, the modified Ti- or Ri-plasmid can be used as a vector for the transfer of the gene construction according to the invention into a suitable plant cell.

The vir region causes transfer of the T-DNA region of Agrobacterium to the genome of the plant cell irrespective of whether the T-DNA region and the vir region are present on the same vector or on different vectors within the same Agrobacterium cell. A vir region on a chromosome also induces the transfer of the T-DNA from a vector into a plant cell.

Within the scope of this invention, therefore, there is preferably used a system for transferring a T-DNA region of Agrobacterium into plant cells, in which system the vir region and the T-DNA region are located on different vectors. Such a system is known as a "binary vector system", and the Vector containing the T-DNA is accordingly designated a "binary vector".

Any T-DNA-containing vector that can be transferred into plant cells and permits selection of the transformed cells is suitable for use within the scope of this invention.

Especially preferred within the scope of this invention is a shuttle vector that contains the chimaeric genetic construction according to the invention cloned in between the left border sequence (LB) and the right border sequence (RB) and that is capable of stable replication both in E. coli and in A. tumefaciens.

Using newly developed transformation techniques, it has also become possible to transform in vitro plant species that are not natural host plants for Agrobacterium. For example, monocotyledonous plants, especially the cereal species and various grasses, are not natural hosts for Agrobacterium.

However, it has become increasingly evident that monocotyledons can also be transformed using Agrobacterium, so that, using new experimental formulations that are now becoming available, cereals and grass species are also amenable to transformation [Grimsley NH et al (1987)].

Preferred within the scope of this invention is so-called leaf disk transformation using Agrobacterium [Horsch et al (1985)]. Sterile leaf disks from a suitable target plant are incubated with Agrobacterium cells containing one of the chimaeric gene constructions according to the invention, and are then transferred into or onto a suitable nutrient medium. Especially suitable, and therefore preferred within the scope of this invention, are LS media that have been solidified by the addition of agar and enriched with one or more of the plant growth regulators customarily used, especially those selected from the group of the auxins consisting of a-naphthylacetic acid, picloram, 2,4,5-trichlorophenoxyacetic acid, 2,4-dichlorophenoxyacetic acid, indole-3-butyric acid, indole-3-lactic acid, indole-3-succinic acid, indole-3-acetic acid and p-chlorophenoxyacetic acid, and from the group of the cytokinins consisting of kinetin, 6-benzyladenine, 2-isopentenyladenine and zeatin. The preferred concentration of auxins and cytokinins is in the range of from 0.1 mg/l to 10mg/l.

After incubation for several days, but preferably after incubation for 2 to 3 days at a temperature of from 20°C to 40°C, preferably from 23°C to 35°C and more especially at 25°C and in diffuse light, the leaf disks are transferred to a suitable medium for the purpose of shoot induction. Especially preferred within the scope of this invention is an LS medium that does not contain auxin but to which a selective substance has been added for the selection of the transformants. The cultures are kept in the light and are transferred to fresh medium at suitable intervals, but preferably at intervals of one week. Developing green shoots are cut out and cultured further in a medium that induces the shoots to form roots. Especially preferred within the scope of this invention is an LS medium that does not contain auxin or cytokinin but to which a selective substance has been added for the selection of the transformants.

In addition to Agrobacterium-mediated transformation, within the scope of this invention it is possible to use direct transformation methods for the insertion of the gene constructions according to the invention into plant material.

For example, the genetic material contained in a vector can be inserted directly into a plant cell, for example using purely physical procedures, for example by microinjection using finely drawn micropipettes [Neuhaus et al (1987)] or by bombarding the cells with microprojectiles that are coated with the transforming DNA ["Microprojectile Bombardment"; Wang Y-C et al (1988)].

Other possible methods for the direct transfer of genetic material into a plant cell comprise treatment of protoplasts using procedures that modify the plasma membrane, for example polyethylene glycol treatment, heat shock treatment or electroporation or a combination of those procedures [Shillito et al (1985)].

In the electroporation technique, plant protoplasts together with plasmids that contain the hybrid gene construction are subjected to electrical pulses of high field strength. This results in a reversible increase in the permeability of biomembranes and thus allows the insertion of the plasmids. Electroporated plant protoplasts renew their cell wall, divide and form callus tissue. Selection of the transformed plant cells can take place with the aid of the above-described phenotypic markers.

A further method for the direct introduction of genetic material into plant cells which is based on purely chemical procedures and which enables the transformation to be carried out very efficiently and rapidly is described in Negrutiu I et al (1987).

Also suitable for the transformation of plant material is direct gene transfer using co-transformation (Schocher RJ et al 1986).
Co-transformation is a method that is based on the simultaneous taking up and integration of various DNA molecules (non-selectable and selectable genes) into the plant genome and that therefore allows the detection of cells that have been transformed with non-selectable genes.

The list of possible transformation methods given above by way of example is not claimed to be complete and is not intended to limit the subject of the invention in any way.

Those cell clones which contain the hybrid gene construction incorporated in their genome are selected using customary selection, screening and detection methods and are used for the regeneration of transgenic plants.

The regeneration of protoplasts kept in culture to form whole plants is described, for example, in Potrykus I and Shillito RD (1986).

The regeneration processes differ from one plant species to another. In general, however, protoplasts in one of the known culture media are stimulated to divide and form cell walls. There are finally formed callus cultures which can be induced to form roots or shoots by treatment with specific active agents, for example auxins and cytokinins.

The plantlets so obtained can then be transferred to soil and cultivated further in the same manner as normal seedlings.

Efficient regeneration depends especially upon the medium, the genotype and the previous history of the culture. If these three variables are adequately controlled, the regeneration is completely reproducible and repeatable.

The regenerated transgenic plants, which contain a structural gene, expressible in the plant cell, of the above-described hybrid gene construction as an integral component of the plant genome, can be propagated vegetatively, preferably in the form of sterile shoot cultures.

The stable integration of an operative expressible gene into the plant genome of the regenerated transgenic plants is verified by reference to the mitotic stability of the integrated gene and on the basis of its behavior as Mendelian characteristic during meiosis and using Southern blot analysis (Southern EM, 1975).

The broad concept of this invention therefore also includes transgenic plant material, selected from the group consisting of protoplasts, cells, callus, tissues, organs, seeds, embryos, pollen, ovules, zygotes etc., including whole plants, but especially whole, fertile plants, that has been transformed by means of the processes described above and contains the recombinant DNA according to the invention in expressible form, and processes for the preparation of said transgenic plant material.

The present invention therefore relates also to the production of recipient plants that at least contain the said structural gene incorporated in their genome.

Preferred are female and/or male sterile plants comprising a structural gene which encodes for the production of a toxin gene, operably linked to a plant specific promoter that is capable of directing the expression of the associated toxin gene in the reproductive tissue, preferably in the anther and/or pistil, of a recipient plant.

Especially preferred are plants wherein said structural gene is genetically linked with a gene capable of imparting herbicide tolerance, preferably with a gene capable of imparting herbicide tolerance to one or more herbicides selected from the group consisting of hygromycin, kanamycin, phosphinotricin, asulam, sulfonylureas, glyphosate, bromoxymil and imidazolinones.

A more complete understanding of the present invention may be had by reference to the following figures and examples which are presented to exemplify, not limit, the present invention. In the figures;
Figure 1 is a restriction map of the EcoR1 fragment containing the S13 SLG gene and showing the derivation of the 3 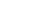 probe specific to the SLG gene. The stippled box represents the SLSG protein-coding region. The black bar indicated by the arrowhead corresponds to the 3 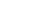 untranslated region from which the gene-specific probe is derived. B= BamH1; E = EcoR1; H = HindIII; S = Sal1; X = Xhol.
Figure 2 is a restriction map of genes SLG-8, SLG-14, SLG-22 and SLG-2. B = BamH1; E = EcoR1; H = HindIII; Sp = SphI; St = Sst1; X = Xbal; K = KpnI, and the orientation is in the 5 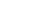 ----- 3 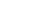 direction.
Figure 3 is a map of SLR2-cDNA isolated from 2 and S15 genotypes. B = BamH1; Bg = Bg12; E=EcoR1; and St= Sst1.
Figure 4 is a summary of the histochemical detection of GUS activity in the anther and stigma of transgenic Arabidopsis.

### NON-LIMITING EXAMPLES:

### General recombinant DNA techniques:

Since many of the recombinant DNA techniques employed in this invention are a matter of routine for the person skilled in the art, it is better to give a short description of these generally used techniques here rather than to describe them every time they occur. Except where there is a specific indication to the contrary, all these procedures are described in the Maniatis et al (1982) reference.

### A. Cleaving with restriction endonucleases

A reaction batch typically contains about 50 to 500 µg/ml of DNA in the buffer solution recommended by the manufacturer, New England Biolabs, Beverly, MA.. 2 to 5 units of restriction endonucleases are added for each µg of DNA and the reaction batch is incubated for from one to three hours at the temperature recommended by the manufacturer. The reaction is terminated by heating at 65°C for 10 minutes or by extraction with phenol, followed by precipitation of the DNA with ethanol. This technique is also described on pages 104 to 106 of the Maniatis et al (1982) reference.

### B. Treatment of the DNA with polymerase in order to produce blunt ends

50 to 500 µg/ml of DNA fragments are added to a reaction batch in the buffer recommended by the manufacturer, New England Biolabs. The reaction batch contains all four deoxynucleotide triphosphates in concentrations of 0.2 mM. The reaction takes place over a period of 30 minutes at 15°C and is then terminated by heating at 65°C for 10 minutes. For fragments obtained by cleaving with restriction endonucleases that produce 5'-projecting ends, such as EcoRI and BamHI, the large fragment, or Klenow fragment, of DNA polymerase is used. For fragments obtained by means of endonucleases that produce 3'-projecting ends, such as PstI and SacI, the T4-DNA polymerase is used. The use of these two enzymes is described on pages 113 to 121 of the Maniatis et al (1982) reference.

### C. Agarose gel electrophoresis and purification of DNA fragments from gels

Agarose gel electrophoresis is carried out in a horizontal apparatus, as described on pages 150 to 163 of the Maniatis et al reference. The buffer used is the tris-borate buffer described therein. The DNA fragments are stained using 0.5mg/ml of ethidium bromide which is either present in the gel or tank buffer during electrophoresis or is added after electrophoresis. The DNA is made visible by illumination with long-wave ultraviolet light. If the fragments are to be separated from the gel, an agarose is used that gels at low temperature and is obtainable from Sigma Chemical, St. Louis, Missouri. After the electrophoresis the desired fragment is cut out, placed in a plastics test tube, heated at 65 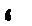 C for about 15 minutes, extracted three times with phenol and precipitated twice with ethanol. This procedure is slightly different from that described by Maniatis et al (1982) on page 170. As an alternative, the DNA can be isolated from the agarose with the aid of the Geneclean kit (Bio 101 Inc., La Jolla, CA, USA).

### D. Addition of synthetic linker fragments to DNA ends

If it is desired to add a new endonuclease cleavage site to the end of a DNA molecule, the molecule is optionally first treated with DNA-polymerase in order to produce blunt ends, as described in the above section. About 0.1 to 1.0 µg of this fragment is added to about 10 ng of phosphorylated linker DNA, obtained from New England Biolabs, in a volume of 20 to 30 µl with 2 µl of T4 DNA ligase from New England Biolabs, and 1 mM ATP in the buffer recommended by the manufacturer. After incubation overnight at 15°C, the reaction is terminated by heating at 65°C for 10 minutes. The reaction batch is diluted to about 100 µl in a buffer appropriate for the restriction endonuclease that cleaves the synthetic linker sequence. About 50 to 200 units of this endonuclease are added. The mixture is incubated for 2 to 6 hours at the appropriate temperature, then the fragment is subjected to agarose gel electrophoresis and purified as described above. The resulting fragment will then have ends with endings that were produced by cleaving with the restriction endonuclease. These ends are usually cohesive, so that the resulting fragment can then readily be linked to other fragments having the same cohesive ends.

E. Removal of 5'-terminal phosphates from DNA fragments

During the plasmid cloning steps, the treatment of the vector plasmid with phosphatase reduces the recircularisation of the vector (discussed on page 13 of the Maniatis et al reference). After cleavage of the DNA with the correct restriction endonuclease, one unit of calf intestinal alkaline phosphatase obtained from Boehringer-Mannheim, Mannheim, is added. The DNA is incubated at 37°C for one hour and then extracted twice with phenol and precipitated with ethanol.

### F. Linking of the DNA fragments

If fragments having complementary cohesive ends are to be linked to one another, about 100 ng of each fragment are incubated in a reaction mixture of 20 to 40 µl containing about 0.2 unit of T4 DNA ligase from New England Biolabs in the buffer recommended by the manufacturer.
Incubation is carried out for 1 to 20 hours at 15°C. If DNA fragments having blunt ends are to be linked, they are incubated as above except that the amount of T4 DNA ligase is increased to 2 to 4 units.

### G. Transformation of DNA into E. coli

E. coli strain HB101 is used for most of the experiments. DNA is introduced into E. coli using the calcium chloride process, as described by Maniatis et al (1982), pages 250 and 251.

### H. Screening of E. coli for plasmids

After transformation, the resulting colonies of E. coli are tested for the presence of the desired plasmid by means of a rapid plasmid isolation process. Two customary processes are described on pages 366 to 369 of the Maniatis et al (1982) reference.

### I. Large-scale isolation of plasmid DNA

Processes for the isolation of plasmids from E. coli on a large scale are described on pages 88 to 94 of the Maniatis et al (1982) reference.

### J. Cloning in M13 phage vectors

In the following description it is to be understood that the double-strand replicative form of the phage M13 derivatives is used for routine processes, such as cleaving with restriction endonuclease, linking etc..

Unless there is a specific indication to the contrary, enzymes can be obtained from Boehringer, Biolabs (BRL). They are used in accordance with the manufacturer's instructions unless otherwise indicated.

### K. Southern blot analysis

The extracted DNA is first treated with restriction enzymes, then subjected to electrophoresis in a 0.8 % to 1 % agarose gel, transferred to a nitrocellulose membrane [Southern EM (1975)] and hybridized with the DNA to be detected which has previously been subjected to nick-translation (DNA-specific activities of 5 x 10⁸ to 10 x 10⁸ cpm/µg). The filters are washed three times for 1 hour each time with an aqueous solution of 0.03M sodium citrate and 0.3M sodium chloride at 65°C. The hybridized DNA is made visible by blackening an X-ray film over a period of 24 to 48 hours.

### L. Western blot analysis

After SDS-polyacrylamide gel electrophoresis, the proteins are transferred electrophoretically to a nitrocellulose or nylon filter. This filter is then first pre-treated with a blocking agent (for example 5 % skim milk powder in PBS: in the following referred to as milk/PBS). The filter is then incubated for several hours with an antiserum that reacts with the compound to be detected. The filter pre-treated in this manner is washed several times with milk/PBS and then incubated with a commercially available secondary antibody that is coupled to an enzyme [for example peroxidase-coupled goat anti-rabbit antibody (BIORAD), 1:2000 diluted in milk/PBS]. The filter is again washed in PBS and then stained with chloronaphthol and hydrogen peroxide in accordance with the manufacturer's [BIORAD] instructions. Further details are given in Sambrook et al (1989).

### M. Northern blot analysis

Total RNA can be prepared in accordance with the method described in Logemann et al (1987), but it is advantageous to introduce an additional precipitation step with 2M LiCl in order to eliminate any residues of contaminant DNA. The RNA (0.4 mg or 4 mg) is then fractionated by electrophoresis in a 1 % agarose gel containing 6.7 % formaldehyde [Maniatis et a (1982)]. The individual fractions are transferred to 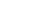 Zeta-Probe 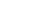 membranes [BIORAD] and hybridized with a ³²P-labelled probe. The size of the hybridizing DNA can be estimated by reference to the standard run at the same time.

### Example I: Vector construction for transformation of self- incompatibility into plants

### (a) Construction of pCIB 542, an agropine VIR helper plasmid bearing a spectinomycin drug resistance gene in the place of the T-DNA

The Ti plasmid, pTi Bo 542 [Sciaky, D., Montoya, A.L. & Chilton, M.-D. (1978)], is of interest because Agrobacteria bearing this Ti plasmid are able to infect the agronomically important legumes alfalfa and soybean [Hood, E.E., et al (1984)]. The construction of a pTi Bo 542 derivative deleted of the T-DNA has been described [Hood, E.E., et al (1986)]. In this construction, named EHA 101, the T-DNA is replaced by the a kanamycin drug resistance gene.
A derivative of EHA 101 having the kanamycin drug resistance gene replaced by a spectinomycin drug resistance gene is constructed as follows: The plasmid p pi delta 307 [Hood, E.E., et al (1986)] has a 1.7 kb region of homology to the left side of Bam 5 of pTi Bo 542 [Hood, E.E., et al (1984)] and an 8 kb region of homology to the right side of Bam2a of pTi Bo 542, separated by a unique EcoR1 site. The plasmid pMON30/SR20, which has been deposited under the Budapest Treaty with ATCC in Rockville, Maryland and is designated with accession number 67113, bears the spectinomycin/streptomycin [spc/str] drug resistance gene from Tn7 [Hollingshead, S. and Vapnek, D. (1985)] pMon 30 is digested with EcoR1, the 5.5 kb fragment containing the spc/str gene isolated from an agarose gel, and ligated into EcoR1-restricted p pi delta 307. The desired recombinant is selected as a spectinomycin resistant (50 µg/ml) tetracyline resistant (10 µg/ml) transformant of E. coli HB101.

Plasmid DNA is introduced into agrobacteria A136/EHA101 by transformation. The desired transformant is selected by its streptomycin-resistant tetracycline-resistant kanamycin-resistant phenotype. Homogenotes [Matzke, A.J.M. & Chilton, M.-D. (1981)] of EHA101 and the spectinomycin plasmid are selected after conjugal introduction of the eviction plasmid R751-pMG2 [Jacoby, G. et al (1976)] and selection on gentamycin (50 µg/ml) and spectinomycin. The desired homogenote has a gentamycin-resistant spectinomycin-resistant tetracycline-sensitive kanamycin-sensitive phenotype. The structure of the resulting plasmid is confirmed by Southern analysis.

A derivative cured of the eviction plasmid is then selected by its drug resistance phenotype of gentamycin sensitivity. This selection is confirmed by Southern analysis.

### (b) Construction of transformation vectors

The Brassica gene used in the transformation according to one embodiment of the present invention is designated SLG-13. It encodes the S-locus specific glycoprotein (SLSG) and is isolated from Brassica oleracea S₁₃ homozygote. The method for the isolation of this gene is described in Nasrallah et al (1988). SLG-13 is contained on an 11 kilobase (kb) EcoR1 restriction fragment which contains the SLSG-coding region and approximately 4.2 kb of 5' and 5.0 kb of 3' flanking sequence. (See Figure 1).

This EcoR1 fragment is inserted into the multiple cloning site of the binary vector pC1B10 [Rothstein et al (1987)] which carries a chimeric kanamycin resistance gene for selection of transformed plants. The recombinant vector pH1 [Moore HM and Nasrallah JB (1990)] or SLG-13/pCIB10 carrying the S₁₃ gene is mobilized into Agrobacterium tumefaciens strain pC1B542/A136 [a derivative of strain EHA101 described in Hood et al (1986); see example I(b)] by triparental mating according to standard published procedures [Rogers SG et al, (1988)].

Transformation vectors carrying SLG genes are also constructed in the Ti plasmids pBI 121 [which is commercially available from Clonetech Laboratories] and pBIN 19 [Jefferson RA et al (1987); Bevan M (1984)]. Plant transformation with these vector has also been successful.

Transformation of Brassica is performed according to a method used in the related crucifer Arabidopsis with a mixture of A. tumefaciens cells harboring the SLG13/pCIB10 construct and of root-inducing plasmid from the wild type A. rhizogenes strain A4 [Van Sluys et al (1987)]. Tissue explants of B.napus var. "Westar" are obtained and treated essentially as described by Fry et al (1987)]. In our experience, Brassica transformation is inefficient. Following selection on kanamycin, one kanamycin-resistant primary transformant, plant17B-1, is obtained and analyzed for the integration and expression of the S13 transgene.

More specifically, this transformation is performed according to the procedure in Example II.

### Example II: Transformation of Brassica napus

Terminal internodes from a flowering stem of Brassica napus var/ "Westar" are removed and surface sterilized in 70 % (v/v) ethanol for 1 minute, 2 % (v/v) sodium hypochlorite for 20 minutes, and followed by a rinse, three times, in sterile distilled water. The sterilized stems are then cut into 5mm discs and dipped into an Agrobacterium cell mixture for 1 minute. The discs are then removed, blotted dry on sterilized filter paper, and placed over a feeder layer of tobacco cells plated onto a co-cultivation medium consisting of MS salts and vitamins, 3 % sucrose, 1 mg/1 NAA, 0.5 mg/1 BAP, and 0.8 % Agar. Following co-cultivation for two days in the dark at 28°C, the discs are transferred to culture plates containing the same medium supplemented with 500 mg/l carbencillin to inhibit bacterial growth and 50 mg/l kanamycin to select for transformation with the SLG-13/pC1B10 construct. The plates are cultured at 25°C under cool white light, and the stem discs are transferred to fresh selective media every two weeks. Kanamycin-resistant plantlets are selected from the culture plates and transferred to rooting medium. After roots developed, the plantlets are potted in soil, grown to maturity, and analyzed for successful transformation.

As part of the analysis of transformed plants, the stable integration of the SLG gene in transformed Brassica napus is examined. In view of the presence in the B. napus genome of several copies related to the SLG gene, the S13 transgene is most easily visualized by hybridization to the gene-specific probe derived from the 3 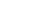 untranslated region of SLSG-cDNA (Figure 1). DNA blot analysis (according to Example III) with this probe revealed the presence of the 11 kb EcoR1 fragment in DNA from 17B-1 but not in DNA from the untransformed "Westar" recipient. Thus no gross rearrangements of the introduced fragment had occurred during transformation and regeneration.

### Example III: DNA blot analysis

Nuclear DNA is prepared from leaf tissue according to published techniques [see, for example, Bingham PM et al (1981)]. Restriction fragments are fractionated on 0.9 % agarose and transferred to Gene Screen Plus membrane [Gene Screen Plus is sold by Dupont/NEN, Dupont Company, Biotechnology Systems, Wilmington, Delaware, USA]. The blot is prehybridized and hybridized at 65°C in 10 % (w/v) dextran sulfate, 330 mM sodium phosphate pH 7.0, 10 mM EDTA, 5 % (w/v) sodium dodecyl sulfate and salmon testes DNA [Sigma Chemical Company, St. Louis, Missouri, USA] at 100 µg/ml. Hybridization is with the 3' probe labeled with ³²P by random priming. The blot is washed in 0.3 M NaCl, 40 mM Tris-HCl pH 7.8, 2 mM EDTA and 0.5 % (w/v) sodium dodecyl sulfate at 65°C, and exposed to Kodak XAR-5 film at -70°C with an intensifying screen.

The stable integration of the S13 gene into the genome of the primary transformant is demonstrated by the analysis of progeny plants obtained by selfing and by backcrossing to the "Westar" line. DNA is isolated at the seedling stage from twenty-one selfed progeny plants and twenty backcross progeny plants. All twenty-one selfed plants are shown by DNA blot analysis to carry the S13 gene, indicating integration at more than one site in 17B-1. This conclusion is supported by the segregation of kanamycin-resistance, a trait for which a larger population is examined. Out of 72 selfed progeny plants assayed, 4 plants are kanamycin-sensitive, giving a ratio with close fit into the 1:15 (sensitive:resistant) ratio expected for two integration events. Furthermore, among 20 progeny plants derived from backcrossing 17B-1 to "Westar", four plants are shown to lack the S13 restriction fragment on DNA blots and to have no detectable SLSG in the stigma. Here again, these numbers approximate the 3:1 backcross ratio expected for two integration events for transforming DNA. The Mendelian ratios obtained in the backcross of 17B-1 to "Westar" also indicate that the ploidy level is correctly maintained during tissue culture.

Consistent with the interpretation of two integration sites are the results of the analysis of restriction digests of genomic DNA isolated from the recipient "Westar" line, the transgenic plant and four of its selfed progeny. Following digestion with EcoR1, the DNA from all four progeny plants is shown to contain the 11 kb fragment carrying the S13 gene. After digestion with HindIII, a restriction enzyme which cuts once within the 11 kb EcoR1 fragment (see Figure 1), two restriction fragments are detected in the transgenic plant and its progeny, and are missing in "Westar" DNA, again consistent with two sites of integration of the S13-containing restriction fragment.

Plant 17B-1 exhibited phenotypic features characteristic of Ri-transformants namely wrinkled leaves, extruded stigmas and loss of apical dominance. All twenty-one progeny plants derived from 17B-1 exhibited the wrinkled-leaf phenotype at the seeding stage. Thus Ri DNA had stably integrated into the 17B-1 genome. In the population of 20 plants obtained by backcrossing 17B-1 to "Westar", two plants are recovered which had normal leaves and are thus apparently free of Ri plasmid. Based on DNA blot hybridization, these two plants are shown to carry the S13 gene, indicating that the Ri and Ti plasmids had inserted on different chromosomes, and that in future generations transgenic plants cured of Ri plasmid can be recovered.

The expression of the S13 gene in the transgenic Brassica napus plants is also studied to confirm that authentic SLSG is being synthesized by the plant. This is possible because the SLSG encoded by the SLG gene in the B. oleracea S13 homozygote (the donor plant) has recently been shown to react with MAb/H8, a monoclonal antibody raised against purified SLSG and specific for a protein epitope of SLG6 [Kandasamy MK et al (1989)]. Stigmas of the "Westar" recipient plant on the other hand are not known to synthesize SLSG, and in any case, they lack a protein with the electrophoretic properties of S13-SLSG, and show no crossreactivity with anti-SLSG antibody. Electrophoretic (according to Example IV) and immunoblotting (according to Example V) analysis of tissue extracts from plant 17B-1, showed the presence of S13-SLSG in stigmas. No antigenic proteins are detected in the style, ovary or leaf. Furthermore, in 17B-1 stigmas, a protein band with the same mobility as S13-SLSG is evident following sodium dodecyl sulfate-polyacrylamide gel electrophoresis (SDS-PAGE), immunoblotting of 17B-1 stigma extracts revealed a pattern characteristic of SLSG and consisting of a cluster of three species with a molecular weight identical to that of S13-SLSG. Stigmas from twenty different inflorescences of 17B-1 are tested by immunoblotting following SDS-PAGE and found to produce SLSG, indicating that the plant is not chimeric.

### Example IV: Protein gel electrophoresis

Stigmas are excised into liquid nitrogen, pulverized in microcentrifuge tubes with an adapted Teflon tissue homogenizer and resuspended in glass-distilled water for isoelectric focusing (IEF) and in 10 mM Tris-HCl, pH 7.2 for SDS-PAGE. The extracts are clarified by centrifugation and total protein content is determined using the Coomassie protein assay (Pierce). Samples containing 50 µg of total protein are subjected to IEF for 2hours on pH 3.5-9.5 polyacrylamide gel slabs. For SDS-PAGE, samples are mixed with an equal volume of a solution containing 160 mM Tris-HCl pH 6.8, 2 % (w/v) SDS, 3 % (w/v) dithiothreitol, 20 % (v/v) glycerol and separated on 10 % (w/v) polyacrylamide gels according to Dreyfuss et al (1984). Molecular weight markers are a mixture of six reference proteins.

### Example V: Immunoblotting

Following electrophoresis, transfer to nitrocellulose is by capillary action for IEF gels and by electroblotting according to published techniques for SDS gels [Towbin H et al (1975)]. For immunostaining, the nitrocellulose membranes with bound protein are blocked with 1 mg/ml BSA in 10 mM Tris-HCl pH 7.2, 0.9 M NaCl and 0.05 % (v/v) Tween-20. The membranes are then treated with MAb/H8, a monoclonal antibody produced against purified SLSG and directed against a protein epitope of SLSG, followed by a treatment with alkaline phosphatase-conjugated anti-mouse IgG (Promega). Immunoreactive components are visualized using BCIP/NBT as substrate.

The level of SLSG produced by 17B-1 is determined as a function of stigma development and relative to that produced in stigmas of the B. oleracea S13 homozygote. For developmental analysis, aliquots containing equivalent amounts of total stigma protein extracted from developing flower buds and flowers are spotted onto nitrocellulose, the blots are stained with MAb/H8 and quantitated by densitometry. Data collected indicates that the transgene-encoded SLSG is detected at three days prior to anthesis, its level increased approximately 7-fold as the stigma matured, and reached a maximum at one day prior to anthesis.

For the relative quantitation of SLSG produced by 17B-1 and by the B. oleracea S13 homozygote, aliquots of a dilution series of total stigma protein extracted from open flowers are used as described in Example VI. The data compiled from different extractions and dilution series indicated that 17B-1 stigmas contained only 60 + 11 % of the level of SLSG found in open flower stigmas of the B. oleracea S13 homozygote.

### Example VI: Quantitative analysis of SLSG

For quantitative analysis of SLSG antigen, the total soluble protein content of stigma extracts is measured with the Coomassie reagent (Pierce). For developmental analysis, equivalent amounts of total protein isolated from stigmas at different stages of development are used. In order to determine the relative amounts of SLG in 17B-1 and the B. oleracea S13 homozygote, a dilution series of total stigma proteins is prepared, and aliquots containing amounts of total protein ranging from 0.001 µg to 1 µg are spotted onto a nitrocellulose membrane using the Hybri-slot apparatus. Following immunostaining, densitometric scanning is performed with a spectrophotometer equipped with gel transport and film holder accessories.

The papillar-cell specific expression of the SLSG gene is transgenic Brassica napus is also studied. The spatial distribution of SLSG in transgenic stigmas is analyzed by indirect immunogold labeling of fixed sections using light and electron microscopy. Of the various cells of the stigma only the papillar cells of 17B-1 stigmas stained positively for SLSG following silver enhanced immunogold staining with MAb/H8. The underlying cells of the stigma and cells of the style failed to stain. Comparable sections from "Westar" stigmas which are subjected to the same staining and development procedures failed to stain with the antibody, and are used as controls for both light and electron microscopic immunolocalization. At the ultrastructural level, indirect immunogold labeling resulted in the absence of gold particles over sections prepared from an untransformed "Westar" plant. In plant 17B-1, only the papillar cells of the stigma showed labeling; the highest concentration of gold particles is observed over the wall of these cells. A few gold particles are also detected over the cytoplasmic membranes. Immunolabeling of 17B-1 stigma sections consistently resulted in a lower density of gold particles as compared to the B. oleracea S13 homozygote.

The fertility of 17B-1 gametes is assayed in reciprocal crosses to "Westar" and other lines by removing pollen from newly dehisced anthers and applying it to the stigmas of the female plant. The pollinated stigmas are stained with decolorized aniline blue and pollen development is monitored by fluorescent microscopy. It is found that, when used as the male parent, 17B-1 had fully functional pollen which produced normal seed set of 20-30 seed per silique. However, the reciprocal crosses revealed some ovule sterility and resulted in only 3-4 seeds per silique on average.

The compatibility/incompatibility phenotype of the transgenic plant is analyzed by microscopic monitoring of pollen tube behavior following pollination. In this assay, the germination and growth of a large number of pollen tubes into the stigma is indicative of a compatible interaction, while the absence of developed pollen tubes is indicative of an incompatible interaction. Self-pollinations and reciprocal cross-pollinations to the "Westar" cultivar are performed, and pollen tube development at the stigma surface is monitored by fluorescence microscopy at different times ranging from 1 to 12 hours after pollination. The determination of compatibility relationships with the B. oleracea S13 homozygote is complicated by the factor of inter-specific incompatibility. Table 1 shows that plant 17B-1 is reciprocally cross-compatible with the B. napus "Westar" cultivar based on the dense pollen tube growth into the stigma at delayed invasion of the stigma by pollen tubes. Initially and up to three hours following pollination, only few pollen tubes invaded the stigma. In 10 different pollinations, between 4 and 57 invading pollen tubes are observed, giving an average of 25 + 19 pollen tubes per stigma. At six hours following self-pollination however, confluent pollen-tube growth is observed.

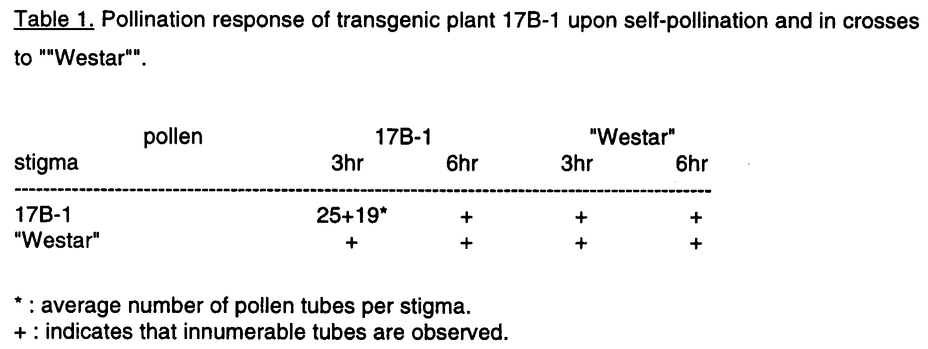

As with the sporophytic Brassica, self-incompatibility in the gametophytic species, Nicotiana alta is also controlled by a single, multiallelic locus, although the gene sequences of between the two are not similar. Whereas the SLG genes in Brassica are expressed specifically in the papillar cell of the stigma, the site of inhibition of pollen germination and pollen tube growth in incompatible pollinations, the S-associated genes in Nicotiana are expressed throughout the pathway of pollen tube growth, in the secretory cells of the stigma, the transmitting tissue of the style and the placenta epidermis of the ovary.

When cloned B. oleracea SLG gene is introduced into the readily transformable and self-compatible species of N. tabacum, the gene expressed itself at the RNA and protein levels. Furthermore, this expression is seen to be developmentally regulated.

### Example VII: Transfer of SLG gene into Nicotiana

### (a) Transgenic plants

Triparental mating is performed using the pH1 binary vector plus pRK2013 [Ditta G et al (1980)] and pCIB542/A136. Leaf strips of axenic plantlets of N. tabacum are transformed using standard methods. Kanamycin resistant shoots are rooted, transplanted to soil and grown to maturity in Environmental Growth System chambers at 70 F with a 16h/8h light/dark cycle. Non-transformed plants regenerated from tissue culture of untreated leaf strips are also grown in these growth chambers.

Tissues for DNA, protein or RNA preparation are dissected from individual transgenic plants, frozen in liquid nitrogen and stored at -70°C until use.

### (b) Stages of floral development

Stages of floral development in N. tabacum are established by collecting bud length data from 10 mm buds to flowers several days after anthesis. On average, buds 5 days (d) before flowering are 13 mm long; buds 3 d before flowering are 25 mm long; buds 2 d before flowering are 40 mm; bud 1 d before flowering are 48 mm. Flowers are 45mm on the first day they opened and are not collected for study after this point.

### (c) DNA and RNA analysis

DNA is prepared from leaves of transgenic plants by a miniprep procedure or by nuclear DNA extraction and CsCl purification. DNA is cut to completion with restriction enzymes, fractionated on 0.6 and 0.7 % (w/v) agarose gels and transferred to Gene-Screen Plus membrane after denaturation. RNA is prepared from leaf, stigma, style and anther tissue by SDS-proteinase K extraction. Total RNA is denatured with glyoxal, electrophoresed on 1 % (w/v) agarose gels and transferred to Gene-Screen membrane.

Concentration of DNA and RNA solutions is determined by spectrophotometry. Hybridization for both DNA and RNA blots is performed at 67°C using a solution containing 10 % (w/v) dextran sulfate, 0.3 M Sodium Phosphate (pH 7.0), 5 % (w/v) SDS, 10mM EDTA, and 0.14 mg/ml denatured salmon sperm DNA. The hybridization probe is a cDNA insert from pBOS13 [Nasrallah JB et al( 1980)] labeled with ³²P-dATP using a random-primer kit. Levels of hybridization signals in different samples are quantitated using a Quick Scan scanning densitometer.

### (d) Protein gel blot analysis

Soluble proteins are extracted from leaf, stigma, style and anther tissue in 100 mM Tris-HCl buffer pH 7.6. Protein concentrations are determined using a bicinchoninic acid/copper sulfate assay system [available from Pierce, P.O. Box 117, Rockford, Illinois, USA]. Prior to fractionation on SDS-PAGE, protein is denatured at 100°C for five minutes in a solution containing 100 mM dithiothreitol, 2 % (w/v) SDS, 80 mM Tris HCl pH 6.8, 10 % (v/v) glycerol and bromphenol blue. Electrophoresis is performed at 20 milliamps and 2°C, using gels of 10 % polyacrylamide and 0.1 % SDS, and electroblotted to nitrocellulose. Protein gel blots are stained with the anti-SLSG monoclonal antibody using the Protoblot Western Blot Alkaline Phosphatase system [Promega, 2800 Woods Hollow Road, Madison, Wisconsin, USA].

### (e) Immunolocalization

S-protein in flowers of transgenic plants is visualized in situ at the light microscope level using silver-enhanced immunogold labeling. Whole immature buds or dissected stigma/style and anther tissue of transgenic plants are fixed for 2 h in 4 % paraformaldehyde in 0.1 M phosphate buffer pH 7.0, dehydrated and embedded in Paraplast Plus media [Monject Scientific, a Division of Sherwood Medical, St. Louis, Missouri, USA]. Ten micron longitudinal sections are cut and mounted using an egg albumin adhesive. Sections are treated sequentially in phosphate buffered saline (10 mM sodium phosphate pH 7.2, 150 mM sodium chloride) containing one of the following: 1) 5 % (v/v) normal goat serum (Sigma) and 5 % (w/v) Bovine Serum Albumin; 2) anti-SLSG antibody, 1:100 in 0.1 % (w/v) BSA; 3) gold-conjugated goat anti-mouse antisera in 0.1 % (w/v) BSA and 5 % (v/v) gelatin. Sections are incubated in each solution for one hour at room temperature; rinses in phosphate buffered saline followed the antibody incubations. Two cycles of silver enhancement are performed using InstenSe II reagents [Janssen Life Science Products, a division of Amersham, 2636 South Clearbrook, Arlington Heights, Illinois, USA].

Twenty-three kanamycin-resistant plants from Example VI are grown to maturity along with nineteen non-transformed plants obtained by leaf disk culture. No morphological differences are observed between the transformed and non-transformed plants. All plants set seed when flowers are selfed by hand pollination and are judged self-compatible on this basis. Twelve kanamycin-resistant plants are selected for further study of the integration and expression of the SLG gene.

DNA purified from leaves of kanamycin-resistant plants is digested with restriction endonucleases, separated by agarose gel electrophoresis, and blotted to nylon membrane. Hybridization analysis is performed using a labeled cDNA probe, pBOS13, which encodes the B. oleracea S13 SLSG. No Hybridization to the pBOS13 probe is detected in DNA from non-transformed N. tabacum under the hybridization conditions used. DNA from two kanamycin-resistant plants did not hybridize to the pBOS13 probe and did not express the SLG gene. Ten kanamycin-resistant plants had DNA fragments which hybridized to the pBOS13 probe and thus are confirmed as transgenic plants.

Transgenic tobacco plants expressed the SLG gene at the RNA and protein levels as determined by RNA gel blot hybridization and binding of an anti-SLSG monoclonal antibody to protein gel blots. Expression of the SLG gene is specific to the stigma and style tissue in flowers and buds of transgenic plants.

Independent transformed plants expressed the SLG gene at different levels, with the highest levels of SLSG expression correlated to multiple sites of insertion of the SLG gene in two transgenic plants. Rearrangement of the introduced SLG gene apparently resulted in a higher level of SLSG in one transgenic plant and a lower level of expression in another. These differences in expression may result from differences in the site of insertion of the rearranged DNA in the N. tabacum genome and/or differences in sequence orientation within the rearranged SLG gene.

The anti-SLSG antibody recognized proteins of transgenic tobacco similar in electrophoretic mobility to the B. oleracea S13 SLSG. In B. oleracea the three molecular weight forms of SLSG are suggested to derive from different posttranslational modifications of the same primary translational product. The results obtained in transgenic tobacco demonstrate that the three forms are derived from a single SLG gene, and that the stylar tissue of tobacco correctly processes the three forms as in B. oleracea.

Maximal levels of SLSG occurred in open flowers of transgenic tobacco, consistent with expression of the self-incompatibility phenotype in B. oleracea and N. alata, and with reported expression of S-proteins in both systems. Expression of the SLG gene in transgenic tobacco differed from B. oleracea in that maximal levels of transcript occurred in open flowers in transgenic plants; in B. oleracea maximal transcript is observed one day before flowering and decreases at anthesis. SLG transcript in stigma and style of transgenic plants is present in the earliest bud stage examined, 5 days before flowering, however, protein blots did not show detectable SLSG until 3 days before flowering. A similar pattern of protein vs transcript accumulation is shown in self-incompatible N. alata. In N. alata, S-associated proteins are not detected at the immature bud or "green bud" stage but are present in styles of mature buds; the transcript of the cloned S-associated gene of N. alata is, however, present at the green bud stage.

The SLG gene is expressed in the stylar transmitting tissue of transgenic tobacco, as demonstrated by silver-enhanced immunogold labeling, consistent with S-associated transcript localization in N. alata. The transmitting tissue is the site where pollen tubes of N. alata and other Solanaceous self-incompatible species encounter a self-incompatibility barrier. In the immunocytochemical analysis of the stigma and style of transgenic N. tabacum, only very low levels of expression of SLSG are detected in the stigmatic epidermis and papillar cells as compared with high levels of SLSG in transmitting tissue is found.

N. tabacum transformed with the B. oleracea SLG gene expressed the gene in a manner more analogous to the gametophytic self-incompatibility system represented by N. alata than to the sporophytic B. oleracea system, where the SLG gene is expressed only in the stigmatic papillar cells. This represents a new level of homology between the two different self-incompatibility systems. The protein-coding regions of the SLG genes of B. oleracea and the S-associated genes of N. alata, as previously noted, do not share significant sequence similarity. Possibly the promoter regions of the B. oleracea and N. alata self-incompatibility genes share sequence motifs which interact with different spatial and temporal regulatory systems presented in the host plants. The different organization of the stigma in the two species (dense papillar cells and "dry" stigma in Brassica, sparse papillar cells and "wet" glandular stigma in Nicotiana) and the different physiology of pollen tube growth in a self-incompatible response may reflect generally different gene regulatory systems in the two species. In some way which is not understood the host plant N. tabacum is able to express a gene for Brassica self-incompatibility in the tissues appropriate to a self-incompatibility response in that host.

Interestingly, the transgenic plants are not self-incompatible as determined by seed set although the SLG gene is expressed. The full complement of genes necessary to effect self-incompatibility may not be present in transgenic tobacco. S-related genes which do not show linkage to the S-locus are expressed in Brassica [Lalonde et al, 1989]. These genes and genes unrelated to the S-multigene family may be essential to the operation of self-incompatibility in Brassica. It is also possible that the molecular structure of B. oleracea SLSG, expressed at the dry stigma surface in Brassica, is not suitable for inhibition of pollen tubes at advanced stages of growth and/or cannot inhibit pollen tube growth when expressed in the glandular matrix of the N. tabacum style. Results of tobacco transformation experiments using the N. alata S-associated genes have not yet been reported but are of interest especially if the N. alata S-coding region is more effective than that of B. oleracea at influencing compatibility in N. tabacum. Transformation of Brassica with N. alata S-associated genes, when available, would allow further comparison between the sites of expression of sporophytic and gametophytic self-incompatibility genes.

As discussed above, we have been successful in introducing cloned S₁₃ and S₈ alleles of the SLG gene into B. napus. In addition, both cloned S₁₃ and S₂₂ alleles have been successfully introduced into Nicotiana, and S alleles have been introduced into Arabidopsis as well.

### Example VIII: Single cross hybrid production

Plants from a self-compatible, herbicide resistant line of plants that are heterozygous for the gene code for an S-locus specific glycoprotein linked with a gene coding for herbicide resistance (S⁺+S⁻) (Line "A") are crossed with a self-fertile, herbicide sensitive line of plants (S⁻S⁻) (Line "B"). This cross produces an F1 hybrid generation that is 50 % self-incompatible, herbicide resistant (S⁺S⁻) and 50 % self-fertile, herbicide sensitive. Pollen from the self-fertile, herbicide sensitive plants will fertilize both self-fertile, herbicide sensitive and self-incompatible, herbicide resistant plants. Thus the hybrid crop will be fully pollinated and will have normal seed set.

Of course, the self-incompatible plants used in the present invention may contain more than one self-incompatible allele linked together.

### Example IX: Production of hybrid seed in the field

Plants from Line A (self-incompatible, heterozygous for the gene coding for an S-locus specific glycoprotein linked with the herbicide resistant gene) and Line B (self-fertile, herbicide sensitive) from Example VII are planted in separate strips. The male Line B is subsequently destroyed by application of the herbicide to which Line A is resistant before harvest. In this instance, all F1 hybrid seed will be derived from harvesting Line A.

Alternatively, Lines A and B may be interplanted and Line B destroyed by subsequent application of herbicide between pollen production and harvest of the seed. Again, all F1 hybrid seed will be derived from harvesting Line A.

### Example X: Maintenance of the heterozygous line

To obtain and maintain the heterozygous self-incompatible, transformed herbicide resistant line of plants described in Example VII, self-incompatible, herbicide resistant plants, either homozygous (S⁺S⁺) or heterozygous (S⁺S⁺) for the self-incompatibility/herbicidal resistant traits, are crossed with self-fertile, herbicide sensitive (S⁻S⁻) plants. The resulting progeny will be a mixture of self-incompatible, herbicide resistant plants (S⁺S⁻) and self-fertile, herbicide sensitive plants (S⁻S⁻).

After germination of the seed from the cross, a herbicide (corresponding to the herbicide to which the self-incompatible are resistant) is applied. The self-fertile, herbicide sensitive plants will be killed, therefore leaving only the heterozygous self-incompatible, herbicide resistant plants (S⁺S⁻) alive.

### Example XI: Single cross hybrid production from lines containing different S-incompatible loci

Line "X" is used to designate a line of plants which are heterozygous, self-incompatible, herbicide resistant (S 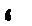 ⁺S⁻), and in which the self-incompatibility and herbicide resistant genes are linked. Line "Y" is used to designate a line of plants which are heterozygous, self-incompatible, herbicide resistant (S"⁺S⁻), and in which the self-incompatibility and herbicide resistance are linked. S 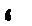 and S" are genes which code for different S-locus specific glycoproteins. The progeny of these lines will comprise mixtures of hybrids containing plants in equal proportions with the following self-incompatibility alleles: (S 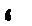 ⁺S^{"+}), (S 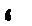 ⁺S⁻), (S"⁺S⁻) and (S⁻S⁻). The (S 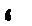 ⁺S"⁺) progeny will be self-incompatible but with the capability of being fertilized by pollen from the (S⁻S⁻) plants. The Line X and Line Y progeny (having the same genes as the parent) are self-incompatible, but cross-fertile with each other as well as capable of being fertilized by the (S⁻S⁻) plants. The (S⁻S⁻) progeny are self-compatible, capable of being fertilized by other like plants, and herbicide sensitive.

### Example XII: Maintenance of lines with different S-incompatible loci

Heterozygous self-incompatible, herbicide resistant Lines X and Y may be maintained in the same manner as Line A is maintained in Example IX.

### Example XIII:

Plants of line X and Y may be interplanted because they are both self-incompatible. It is not necessary to effectively separate the parent lines, as in Example VIII. Also, no herbicide application is necessary.

### Example XIV: Construction of plasmids pMTK8 and pMTK17

An SLG gene promoter fragment is isolated from the SLG-13 gene [Nasrallah JB and Nasrallah ME (1988)]. The oligonucleotid 5 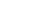 -GAAAAAGGAGGGGATCCAAATGAAAGGCG-3 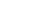 (SEQ ID NO: 7) is used to introduce three nucleotide changes (underlined) in the SLG-13 gene by site-directed mutagenesis [Kunkel TA (1985)]. This mutation generated a BamHI site 8 bp upstream of the SLG-13 ATG codon. The S₁₃ promoter fragment extended from this introduced BamHI site to a natural BamHI site 3.65 kb upstream of the SLG-13 ATG codon.

The 3.65 kb BamHI S₁₃ promoter fragment is fused to the 280 bp SstI-EcoRI fragment isolated from plasmid pBI121 [Jefferson RA et al (1987)] that contains the polyadenylation signal from the nopaline synthase gene of the Agrobacterium tumefaciens Ti plasmid [Bevan MW et al (1983a); Bevan MW et al (1983b)], and these sequences are inserted into the binary vector pBIN19 [Bevan MW (1984)]. A 795 bp BgIII fragment that encodes the diphtheria toxin A polypeptide is isolated from plasmid DT-A [Palmiter RD et al (1987)] and inserted into a BamHI site between the S-₁₃ promoter and the nopaline synthase polyadenylation signal. The resulting plasmid pMKT17, has a kanamycin resistance gene for transformation selection and the S₁₃:DT-A fusion within the T-DNA borders.

An S₁₃:GUS fusion is made by inserting the 3.65 kb BamHI S₁₃ promoter fragment into the BamHI site upstream of the GUS gene in the binary vector pBI101 [Jefferson RA et al (1987)]. The resulting plasmid, pMKT8, has a kanamycin resistance gene for transformation selection and the S₁₃:GUS fusion within the T-DNA borders.

### Example XV Tissue-specific expression of the GUS reporter gene

The resulting vector plasmid, pMKT8 is introduced into Arabidopsis thaliana, a member of the Brassicaceae that has a short generation time, is easily transformed, and in which the self incompatibility trait is not known to occur. However, the rapid flowering of regenerated transgenic plants makes Arabidopsis well suited for the analysis of flower-specific promoters such as those of the S-locus glycoprotein gene. Root segments are inoculated with Agrobacterium cells harboring pMKT8, and transgenic plants are selected based on kanamycin resistance [transgenic plants are produced following infection of Arabidopsis thaliana (L.) Heynh. strain C24 root explants with Agrobacterium tumefaciens as described in PNAS USA 85:5536 (1988); the Agrobacterium stain is pCIB542/A136, a strain derived from helper plasmid pEHA101 containing binary vector pMKT8; transformants are selected, and seeds produced by these plants are germinated on medium containing 50mg/l kanamycin].

Starting with 125 root segments, 20 independent transgenic plants are obtained. Of these, 13 plants exhibited GUS activity in the stigma when assayed by histochemical staining with 5-bromo-4-chloro-3-indolyl glucuronide (X-Gluc) [see R.A.Jefferson et al (1987). This assay involved introducing a solution containing 2mM X-Gluc, 0.1 M sodium phosphate buffer at pH7.0 and 0.5% Triton X-100 into various plant tissues by a brief vacuum infiltration. After an overnight incubation at 37°C, the tissues are rinsed in 70% ethanol and mounted for microscopic observation. A blue staining of the tissues is indicative of GUS activity.

Because the GUS assay is destructive, the flowers of primary transformants (T₁ generation) are saved to ensure seed production. Further studies are carried out on the kanamycin-resistant selfed progenies (T₂ generation) derived from 11 independent GUS-positive transformants.

Seed from the primary transformant plants are plated on kanamycin-containing medium and the number of green kanamycin-resistant (km^{r}) and bleached kanamycin-sensitive (km^{s}) seedlings is determined and tabulated in the following Table.

Segregation analysis and GUS activity in the T₂ progenies of transgenic Arabidopsis.

| T₁ plants | T₂ plants Km^{r} | GUS activity Km^{s} | stigma | anther |
|---|---|---|---|---|
| 1 | 48 | 19^{a} | +++ | - |
| 2 | 89 | 35^{a} | ++++ | ++ |
| 3 | 60 | 7^{b} | +++ | - |
| 4 | 44 | 18^{a} | ++++ | ++ |
| 5 | 13 | 62 | ++++ | + |
| 7 | 126 | 39^{a} | +++ | - |
| 13 | 33 | 10^{a} | +++ | - |
| 16 | 58 | 21^{a} | +++ | - |
| 18 | 108 | 28^{a} | +++ | - |
| 20 | 25 | 14^{a} | ++++ | ++ |
| 23 | 35 | 13^{a} | +++ | - |
| (-) indicates no GUS activity; (+) indicates GUS activity; the number of (+) symbols indicate differences in intensity of GUS staining. | | | | |

| | | | | |
|---|---|---|---|---|
| ^{a} and ^{b} indicate a 3:1 and 15:1 ratios, respectively (significant at the 5% level based on X² tests). | | | | |

For ten transformants, the observed km^{r}:km^{s} segregation ratios are either 3:1 or 15:1. Thus indicating that these plants carried one or two T-DNA integrations, respectively. The ratio for transformant 5 is aberrant, and could not be used to predict the number of T-DNA integrations. The intergration of transforming DNA in several km^{r} T₂ plants is additionally analyzed by DNA blot hybridization to physically characterize the T-DNA insert in GUS positive transformants. A restriction fragment of the expected 3.65 kb size is identified when the 3.65 kb BamHI S-locus glycoprotein gene promoter fragment is used to probe BamHI-digested DNA isolated from various transformants. All transformants contained the predicted 3.65kb S₁₃ promoter fragment; some contained additional bands homologous to the S-promoter probe, suggesting that they contained rearranged T-DNA copies. The pattern of hybridization with the GUS probe indicated that all transformants contained multiple T-DNA copies; in some transformants these copies may have been genetically linked, or some reporter gene fusions may have been inactive as indicated by the segregation of km^{r} found in selfed progeny. No hybridization is observed with DNA obtained from a control untransformed Arabidopsis plant.

All kanamycin-resistant T₂ plants exhibited GUS activity in the pistil. The blue GUS staining is detected in the papillar cells of the stigma surface, is not detected in style or ovary, and increased as the stigma matured. The highest intensity of staining is observed in the progeny from four transformants, specifically plants 2, 4, 5 and 20. In these plants, GUS activity is detected in anthers as well as in stigmas as depicted in the preceding Table. The intensity of staining of anthers varied, and is strongest in the progenies of plants 2, 4 and 20, and weakest in the progeny of plant 5. In the progenies of the remaining 7 transformants, the fusion gene is either not expressed in the anthers or is expressed at a level too low to be detectable under the conditions of the histochemical assay. The variation in different transgenic lines is attributed to effects of position of integration.

The development pattern of S-locus glycoprotein gene promoter activity is also analyzed in progeny plants of transformants 2, 4, 5 and 20. No GUS activity is detected in the leaves, stems, or roots of the transgenic seedling plants. In all transgenic lines, only the stigmas and anthers showed GUS activity. A similar spatial and temporal pattern of reporter gene expression is observed in these lines, and is inherited in the T₃ generation. This pattern is summarized diagrammatically in Fig.4. More specifically, this figure is a summary of the histochemical detection of GUS activity in the anther and stigma tissues of transgenic Arabidopsis as a function of microspore and pollen development. In this figure, "days before anthesis" refers to the age of flower buds; the cross-hatched areas are estimates of the relative staining intensities and are intended to depict the general developmental pattern of expression in diagrammatic form.

As depicted in Figure 4, GUS activity is absent in premeiotic buds and buds of the tetrad stage at 7 days before anthesis. At the early uninucleate microspore stage at 6 days before anthesis, blue staining is apparent in cells of the tapetum, in correlation with the beginning of exine deposition. Neither anthers incubated in the GUS assay buffer without X-Gluc nor anthers of untransformed control plants showed any blue staining, excluding the possibilities of non-specific staining and intrinsic GUS activity. No staining is detected in microspores or mature pollen grains. The intensity of staining in the tapetum is highest at the early uninucleate stage and gradually decreased in buds at 5 days before anthesis which correspond to the late uninucleate microspore stage. Staining disappeared in buds containing binucleate pollen, coincident with tapetal cell degeneration at 4 days before anthesis. At this stage, neither anther nor stigma showed any GUS activity. In subsequent developmental stages, no GUS activity is detected in anther tissue, in isolated mature pollen grains, or in pollen after germination on the stigmas of untransformed plants. However, in buds at 3 days before anthesis (corresponding to the late binucleate pollen stage) faint blue staining could be detected in the stigmatic papillae. Staining of the stigma is clearly visible in buds containing trinucleate pollen at 2 days before anthesis, and is most intense in the open flower. The intensity of GUS staining of the mature stigma is much stronger than that observed in the tapetum of the younger anther.

The temporal regulation of fusion gene expression is further characterized by isolating microspores from buds at different developmental stages. These are double-stained, first with X-Glu to localize GUS activity, and then with the DNA fluorochrome DAPI (4 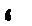 -6-di-amidino-2-phenylindole) which is used at a concentration of 1 µg/ml [see T.Kuroiwa et al (1982)] in a buffer containing 20mM Tris-HCl at pH7.6, 0.25M sucrose, 1mM EDTA, 1mM MgCl₂, 0.1 mM ZnSO₄, 0.1 mM CaCl₂, 0.8mM phenylmethylsulfonylfluoride, 0.05% b-mercaptoethanol, and 1% Triton X-100. The DAPI is used to identify the nuclear condition of the microspore. GUS staining is observed primarily in binucleate pollen grains, although weak staining is occasionally observed in mononucleate microspores. Staining of microspores is not detected until after the initiation of tapetal cell degradation. GUS activity is not detected in the tapetum, or in any other tissue of the anther.

The incidence of GUS staining in pollen of mature flowers is determined by counting the number of grains that are stained blue after incubation with X-Glu. Two plants predicted to have an active T-DNA at one genetic locus showed blue staining in 50% of its pollen grains, and one plant predicted to have an active T-DNA at two loci showed blue staining in 75% of its pollen grains. This incidence of staining is consistent with the observation of gametophytic expression in the haploid pollen grain, rather than sporophytic expression by some other tissue of the anther.

These data resulting from the expression of this reporter gene function have demonstrated that the specific expression of a single S-locus glycoprotein gene promoter can function in two tissue types found in the flower which are involved in the self incompatibility reaction: the papillar cells of the stigma surface and the tapetal cells of the anther. Expression of the S-locus glycoprotein gene promoter is not coordinately regulated in the two cell types, but is temporally separated by a period of approximately 3 days in the development of the flower. This data suggests that a single protein determinant may direct the self incompatibility reaction in these two tissues.

In the stigma, the site and timing of expression coincided with the previously described expression of S-locus glycoprotein genes in Brassica. Arabidopsis is self-fertile genus with no known self-incompatible strains. Nevertheless, the drastic increase in GUS expression in stigmas at one day before anthesis paralleled the acquisition of the self incompatibility response by the developing stigma in self-incompatible Brassica strains. In the anther, GUS expression is observed in the tapetum during a narrow developmental window after meiosis. This result implies that sporophytic control of self incompatibility phenotype in crucifers is based on the expression of the S-locus postmeiotically in the tapetum, rather than on premeiotic expression as has been previously suggested by K.K. Pandy (1960). In comparison with mature stigmas, anthers exhibited only weak staining however. This low level of expression may account for the fact that detection of the S-locus glycoprotein gene product in anthers of self-incompatible Brassica stains has met with little success to date. However, as shown in the present invention, the single S-locus glycoprotein gene has the requisite expression profile for the determination of the pollen-stigma interaction of incompatibility; the novel finding of S-locus glycoprotein promoter activity in the anther supports the hypothesis that sporophytic control of self incompatibility is a result of s-locus glycoprotein gene expression in the tapetum.

### Example XVI Tissue specific expression of a transformed toxin gene

In addition to the reporter gene fusion described above, a second fusion gene construct is made in order to further characterize the sites of S-gene expression as well as to study the function of tissues that express the S-gene. In this construct, the 3.65kb S₁₃ gene promoter fragment is fused to the coding sequence of the Diphtheria toxin A polypeptide in the plant transformation vector pBIN19 [see M Bevan (1984)] to generate plasmid pMKT17. Diphtheria toxin A polypeptide is known to block protein synthesis through ADP-ribosylation of elongation factor II in many organisms, including plants [see R.J. Collier (1977)], and has also been demonstrated to have nuclease activity [see M.P. Chang et a (1989)] These cytotoxic effects allow a single molecule of Diphtheria toxin A polypeptide to cause cell death [see M.Yamizumi et al (1978)], and so Diphtheria toxin A polypeptide can serve as a very sensitive indicator of gene expression. Gene fusions to Diphtheria toxin A polypeptide have been used to genetically direct cell death in a variety of mammalian tissues [see R.R. Behringer et al (1988)].

The S₁₃:Diphtheria toxin A polypeptide toxic gene fusion is introduced into tobacco by Agrobacterium-mediated transformation. Eight independent kanamycin resistant (km^{r}) transformants are obtained and characterized together with eight untransformed control plants. Drastic morphological differences are observed between flowers, but not other tissues, of untransformed controls and six of the eight S₁₃:Diphtheria toxin A polypeptide transformants. The six positive transformants could be divided into two classes based on the severity of the phenotype induced by toxic gene expression. In five Class I transformants, the effects of toxic gene expression are relatively moderate, while in one Class II transformant they are more severe. The differences in phenotype between Class I and Class II transformants is attributed to differences in the level of toxic gene function expression.

As for the S₁₃:GUS fusion described earlier, the effects of S₁₃:Diphtheria toxin A polypeptide fusion expression are apparent in two tissues of the flower, the pistil and the pollen. In Class I transformants, flowers appeared normal externally, and typically reached maturity.

The styles of these flowers are, however, dramatically shorter than those of the control plants. Style length in open flowers of these transformants ranged from 4 to 9mm, as compared with 34 to 36mm in control plants. At the surface of the style, the stigma, there are additional morphological differences between the transformants and controls. The secretory zone of the stigma is ablated in transformants, causing a reduction in size of the stigma surface, and a change in the appearance of this surface tissue. Stigmas of transformants are white, and often contained regions of brown necrotic tissue, while those of control plants are bright green. Additionally, some stigmas of some transformants contained three rather than two lobes. This tri-lobed structure is found throughout the pistil, in the stigma, style and ovary.

A more detailed analysis of the stigma structure is conducted by scanning electron microscopy. As seen when the stigma surface of buds at different developmental stages is analyzed, the effects of toxic gene expression increased as buds aged. Buds at 5 days prior to flowering are nearly normal; the stigma surface had only a slight central indentation which became more severe in older buds, in addition there is a deep furrow in mature buds at 1 day prior to flowering. This is in dramatic contrast to the rounded stigma surface of the untransformed control. The papillar cells at the stigma surface are present in the toxic fusion gene transformants, however the morphology of some papillae is abnormal.

Microscopic analysis of thin sections through the style is used to identify the cell types affected by toxic gene expression. In untransformed tobacco, the transmitting tissue filled the central area of the style. Expression of the S₁₃:Diphtheria toxin A polypeptide fusion resulted in ablation of the transmitting tissue of the style. In mature buds, the transmitting tissue in the region of the style near the stigma is compressed, and ultrastructural analysis showed that cells in this region are dead. In the region of the style near the ovary, the transmitting tissue is completely ablated, and hollow due to the death of the transmitting tissue. The outer parenchymal tissue of the style is unaffected by toxic gene expression. The tissue of the ovary appeared normal when analyzed by light microscopy.

Pollen from untransformed control plants is used to pollinate transformants, and the growth of pollen tubes is analyzed microscopically after staining with aniline blue. Pollen did not germinate on the ablated stigmas of open flowers. Additionally, transformants never set seed through either self-pollination or hand pollination with control pollen, further indicating that ablated pistils are unable to support pollen tube growth. Pollen germination is also analyzed in young buds in which the pistil had only minor morphological differences from untransformed controls. Although pollen did germinate on the stigmas of young buds, pollen tubes are never observed growing.

The buds of Class II transformant are often withered due to abscissions from the flowering stem. Abscission occurred before buds matured, typically by three days prior to flowering, and open flowers are never observed on this plant. The effects of Diphtheria toxin A polypeptide expression on the pistil are more severe than in Class I transformants. Notably, the style is very short (1-2mm), and is not morphologically distinct from the ovary. Additionally, fusion of the carpels is incomplete, and the morphology of the stigma is bizarre. Ovary structure is also altered in this transformant: the placenta is smaller than in controls, the ovules are dead or absent, and there are large open spaces in the locules.

Pollen is also affected by expression of Diphtheria toxin A polypeptide as characterized in Class I transformant. Pollen from open flowers of toxic gene transformants is a mix of normal round grains and small collapsed grains. In transformants, greater than 50% of the pollen grains are small and collapsed, while in untransformed controls less than 15% had this morphology. When pollen from toxic gene transformants is germinated in vitro, only round grains germinated, indicating that the collapsed grains are not viable.

DNA blot hybridization analysis is used to physically characterize the T-DNA insert in Diphtheria toxin A polypeptide transformants. An S₁₃ promoter probe hybridized to the predicted 3.65kb S13 promoter fragment in BamHI-digested DNA of all transformants. The intensity of hybridization to this probe varied in different transformants, and in particular is strongest in the ClassII transformant. The severe phenotype in this transformant may have been the result of multiple T-DNA insertions. Alternatively, the site of T-DNA integration may have affected the level of toxic gene expression.

Thus, as described above, the tissues are affected by toxic gene expression. In the tobacco pistil, the Brassica S-gene promoter is expressed in the secretory zone of the stigma, in the transmitting tissue of the style, and in the placenta of the ovary. S-gene expression is also observed in pollen of transgenic tobacco. Although previous attempts to identify the S-locus glycoprotein gene in pollen of Brassica and transgenic tobacco are unsuccessful, the sensitivity of the GUS histochemical assay and the Diphtheria toxin A polypeptide bioassay allowed detection of these fusion gene products. The results of the present invention clearly indicate that a single S-locus gene may actually direct the incompatibility response in pistil and pollen.

Thus while we have illustrated and described the preferred embodiment of our invention, it is to be understood that this invention is capable of variation and modification and we therefore do not wish to be limited to the precise terms set forth, but desire to avail ourselves of such changes and alternations which may be made for adapting the invention to various usages and conditions. Accordingly, such changes and alterations are properly intended to be within the full range of equivalents, and therefore within the purview of the following claims.

Having thus described our invention and the manner and a process of making and using it in such full, clear, concise and exact terms so as to enable any person skilled in the art to which it pertains, or with which it is most nearly connected, to make and use the same.

### BIBLIOGRAPHY

**Bateman AJ**, Heredity 9: 53 (1955)
**Behringer RR** et al, Genes Dev. 2: 453 (1988)
**MBevan**, Nucleic Acids Res. 12: 8711 (1984)
**Bevan MW** et al, Nucl. Acids Res. 11, 369-385 (1983a)
**Bevan MW** et al, Nature(London) 304, 184-187 (1983b)
**Bingham PM** et al, Cell 25, 693-704 (1981)
**Cashmore A,** "Genetic Engineering of Plants", An Agricultural Perspective, Plenum, New York (1983); pp 29-38].
**Chang** et al, Science 246: 1165 (1989)
**Collier RJ**, "The specificity and action of animal, bacterial and plant toxins", P.Cuatrecasas, Ed. Chapman and Hall, London (1977)
**Dwyer KG** et al, Genome 31: 969 (1989)
**Frank G** et al, Cell, 21: 285-294 (1980)
**Fromm** et al, Proc. Natl. Acad. Sci., USA 82: 5824 (1985)
**Fry** et al, " Transformation of Brassica napus with Agrobacterium tumefaciens based vectors", Plant Cell Rep. 6: 321 (1987)
**Gardner RC** et al, Nucl. Acids Res., 9: 2871-2888 (1981)
**Grimsley NH** et al, Nature 324: 177-179 (1987)
**Heslop-Harrison J**, Nature 218: 90 (1968)
**Heslop-Harrison J**, Ann. Rev. Plant Physiol. 26: 403(1975)
**Hollingshead, S. and Vapnek, D.,** Plasmid 13: 17-30 (1985)
**Hohn T** et al, in: "Molecular Biology of Plant Tumors", Academic Press, New York, pp. 549-560 (1982)
**Hood, EE** et al, Bio/Technology 2: 702-708 (1984)
**Hood, EE** et al, J. Bacteriol. 168: 1291-1301 (1986)
**Horsch RB** et al, Science 227: 1229-1231 (1985)
**Howard** et al, Planta 170: 535 (1987)
**Jacoby, G** et al, J. Bacteriol. 127: 1278-1285 (1976)
**Jefferson RA** et al, EMBO J. 6: 3901-3907 (1987)
**Kandasamy MK** et al, Dev. Biol. 134: 462 (1989)
**Kunkel TA**, Proc. Natl. Acad. Sci., USA 82, 488-492 (1985)
**Kuroiwa T,** et al, Nature 298: 481 (1982)
**Lalonde** et al, Plant Cell 1: 249-258 (1989)
**Mackay GR**, Euphytica 26: 511 (1977)
**Maniatis** et al, "Molecular Cloning", A Laboratory Manual, Cold Spring Harbor Laboratories, 1982
**Matzke, AJM & Chilton M-D** , J. Mol. Appl. Genet. 1: 39-49 (1981)
**Moore HM and Nasrallah DUB,** The Plant Cell 2, 29-38 (1990)
**Morelli** et al, Nature 315: 200 (1985)
**Nasrallah JB** et al, Ann. Rev. Genetics 23: 121 (1989)
**Nasrallah JB** et al, Proc. Natl. Acad. Sci, USA 85: 5551 (1988)
**Negrutiu I** et al, Plant Mol. Biol., 8: 363-373 (1987)
**Neuhaus** et al, Theor. App. Genet., 74: 30-36 (1987)
**Palmiter RD** et al, Cell 50, 435-443 (1987)
**Pandy KK**, Evolution 14: 98 (1960)
**Petit** et al, Mol. Gen. Genet., 202: 388 (1986)
**Potrykus I and Shillito RD**, Methods in Enzymology, Vol 118, Plant Molecular Biology, eds. A and H Weissbach, Academic Press, Orlando, 1986
**Rogers SG** et al, "Use of cointegrating Ti plasmid vectors.", in: Gelvin SB, Shilperoort RA (eds.). Plant Mol. Biol. Manual, Kluwer Academic Publishers (1988)
**Rothstein** et al, Gene 53, 153-161 (1987)
**Schocher RJ** et al, Bio/Technology, 4: 1093-1096 (1986)
**Sciaky, D, Montoya, AL & Chilton, M-D** (1978)
**Shillito** et al, Bio Technology, 3: 1099-1103 (1985)
**Southern** EM, J. Mol. Biol. 98: 503-517 (1975)
**Towbin H** et al, Proc. Natl. Acad. Sci., USA 76: 4350-4354 (1975)
**Spena** et al, EMBO J. 4: 2736 (1985)
**Valvekens D** et al, Proc. Natl. Acad. Sci., USA 85: 5536-5540 (1988)
**Van Sluys** et al, "Studies on the introduction and mobility of the maize activator element in Arabidopsis thaliana and Daucus carota", EMBO J. 6: 3881 (1987)
**Wang Y-C** et al, Plant Mol. Biol., 11: 433-439 (1988)
**Yamizumi M** et al, Cell 15:245 (1978)

## Claims

1. A tissue-specific promoter directing expression of a linked toxin gene in the reproductive tissue of a recipient plant.

2. The promoter according to claim 1 directing expression of the linked toxin gene in the pistil of the recipient plant.

3. The promoter according to claim 2 comprising a region of the SLG-13 gene.

4. The promoter according to claim 3 comprising the region extending from approximately position -17 to approximately position -411 of the SLG-13 gene.

5. The promoter according to claim 4 comprising the region extending from approximately position -150 to approximately position -411 of the SLG-13 gene.

6. A recombinant DNA vector comprising:
(a) the promoter according to any one of claims 1 to 5;
(b) a toxin gene; and
(c) a plant transformation vector.

7. The recombinant DNA vector according to claim 6, wherein the promoter is a pistil-specific promoter sequence of the SLG-13 gene.

8. The recombinant DNA vector according to claim 6, wherein the toxin gene is selected from the group consisting of nuclease genes, proteolytic enzyme genes, lectin genes, protein synthesis inhibitor genes, genes that controll cell division, lytic peptide genes, lipase genes, ribozyme genes, phospholipase genes, genes for enzymes in the pathway for synthesis of a phytohormone and genes for inhibitors of cell wall synthesis.

9. A recombinant DNA molecule comprising the fusion of:
(a) a promoter according to any one of claims 1 to 5;
(b) a toxin gene; and
(c) one or more further structural genes.

10. The recombinant DNA molecule according to claim 9, wherein said structural gene is a reporter gene the expression of which in a transgenic cell is indicative of the incorporation of said toxin gene.

11. The recombinant DNA molecule according to claim 10, wherein said structural gene is a gene capable of imparting herbicide tolerance to a plant.

12. A recombinant DNA transfer vector comprising the recombinant DNA molecule according to any one of claims 9 to 11 incorporated into a plant transformation vector.

13. A method of obtaining reproductive tissue-specific expression of a toxin gene in a plant comprising:
(a) isolating a promoter according to any one of claims 1 to 5;
(b) operably linking said promoter with a toxin gene; and
(c) transforming a plant with said construct.

14. The method according to claim 13, wherein the promoter causes pistil-specific expression of said structural gene.

15. The method according to claims 13 or 14, wherein said structural gene is a toxin gene.

16. A method for obtaining a male and/or female sterile plant which method comprises the following steps:
(a) isolating a promoter according to any one of claims 1 to 5;
(b) operably linking said promoter with a DNA sequence encoding a toxin;
(c) inserting the resulting chimeric toxin gene into the genome of a recipient plant;
(d) allowing the recipient plant to mature and assaying the flower parts of the mature plant for the expression of the toxin gene; and
(e) selecting those plants which show expression of the toxin gene.

17. A female and/or male sterile plant and the asexually derived progeny thereof still containing the trait of male or female sterility, comprising a toxin gene operably linked to a promoter according to any one of claims 1 to 5.
